# EUROPEAN PATENT APPLICATION

(11) **EP 4 732 786 A1**
(43) Date of publication of application: **29.04.2026**
(21) Application number: 24905130.1
(22) Date of filing: 21.11.2024
(51) Int. Cl.: A61B 17/04, A61B 17/06

(54) **SUTURING DEVICE SYSTEM**

(30) Priority: 30.08.2024 CN 202411204094
(71) Applicant: WYTD Medical Technology (Shenzhen) Co., Ltd., Shenzhen, Guangdong 518122 (CN)
(72) Inventor: KANG, Libiao, Shenzhen, Guangdong 518122 (CN); CHEN, Guanlun, Shenzhen, Guangdong 518122 (CN); ZHENG, Zhenkun, Shenzhen, Guangdong 518122 (CN); GONG, Dajia, Shenzhen, Guangdong 518122 (CN)
(74) Representative: Dilg, Haeusler, Schindelmann Patentanwaltsgesellschaft mbH
(86) International application number: PCT/CN2024/133624
(87) International publication number: WO 2026/044953

(57) **Abstract**

A suture system relates to the technical field of medical devices, and comprises a needle ejection device and a suture control device. The needle ejection device includes a needle ejection handle, a guide member, a pushing body and a suture needle. The needle ejection handle includes a needle ejection top cover and a needle ejection bottom cover which are slidably connected. The guide member is located in the needle ejection handle, and the guide member and the needle ejection top cover are fixedly connected. The pushing body and the guide member are movably connected. At least two suture needles are configured, the suture needle is fixed to the pushing body, and the pushing body and the suture needle are arranged in a one-to-one correspondence. The needle ejection top cover is configured to slide relative to the needle ejection bottom cover to drive the guide member to move. The pushing body is configured to move along the guide member to drive the connected suture needle to move. The needle ejection device in the designed suture system can realize both synchronous needle ejection and individual needle ejection, effectively improving the use efficiency and safety of the suture system, improving the puncture effect of the suture needle, and improving the success rate of the operation.

## Description

### CROSS REFERENCE TO RELATED APPLICATION

The present disclosure claims priority to Chinese patent application No. 202411204094.4, filed with the Chinese Patent Office on August 30, 2024, and entitled "A SUTURE SYSTEM", the entire contents of which are incorporated by reference into the present disclosure.

### TECHNICAL FIELD

The present disclosure relates to the technical field of medical devices, and in particular to a suture system.

### BACKGROUND ART

Patent foramen ovale closure can be performed by interventional suturing, with the suture needle passing through the tissue around the foramen ovale for suturing, and then suturing with suture needles.

However, the currently used suturers cannot select the appropriate needle ejection scheme according to the different sizes of the foramen ovale tunnel when suturing the foramen ovale, resulting in unsatisfactory needle puncture results and reduced surgical success rates.

### SUMMARY

The present disclosure provides a suture system, which can improve the puncture effect of the suture needle and increase the success rate of the operation.

In an embodiment of the present disclosure, a suture system is provided, comprising: a needle ejection device and a suture control device; the needle ejection device is configured to push out or retract the suture needle, and the suture control device is configured to push or retract the suture;
the needle ejection device comprises a needle ejection handle, a guide member, a pushing body and a suture needle;
the needle ejection handle comprises a needle ejection top cover and a needle ejection bottom cover which are slidably connected;
the guide member is located in the needle ejection handle, and the guide member is fixedly connected to the needle ejection top cover;
the pushing body and the guide member are movably connected;
at least two suture needles are configured, each suture needle is fixed to a pushing body, and the pushing bodies and the suture needles are arranged in a one-to-one correspondence;
the needle ejection top cover is configured to slide relative to the needle ejection bottom cover to drive the guide member to move; and the pushing body is configured to move along the guide member to drive the connected suture needle to move.

According to the suture system in the above-mentioned embodiment, when suturing the foramen ovale, the needle ejection top cover can slide relative to the needle ejection bottom cover, and the guide member and the multiple suture needles on the guide member are synchronously driven to move synchronously. When a suture needle is in an inappropriate position, there is no need to re-puncture as a whole. The pushing body connected to the suture needle can be used to adjust the inappropriate position of the needle to be ejected separately for puncture again. That is, the needle ejection device in the suture system of the embodiment of the present disclosure can realize both synchronous needle ejection and separate needle ejection, effectively improving the use efficiency and safety of the suture system, improving the puncture effect of the suture needle, and increasing the success rate of the operation.

### BRIEF DESCRIPTION OF DRAWINGS

Figure 1 is a schematic diagram of an exploded structure of a needle ejection device in the embodiment of the present disclosure;
Figure 2 is a schematic diagram of a sectional structure of the needle ejection device in the embodiment of the present disclosure;
Figure 3 is a schematic diagram of a guide member structure in the embodiment of the present disclosure;
Figure 4 is a schematic diagram of an assembly structure of a first pushing body in the embodiment of the present disclosure;
Figure 5 is a schematic diagram of an assembly structure of a second pushing body in the embodiment of the present disclosure;
Figure 6 is a schematic diagram of an assembly structure of a guide member in the embodiment of the present disclosure;
Figure 7 is a schematic diagram of a pressing structure in the embodiment of the present disclosure;
Figure 8 is a schematic diagram of a partial structure of a suture control device in the embodiment of the present disclosure;
Figure 9 is a structural schematic diagram of a suture pushing assembly in the embodiment of the present disclosure;
Figure 10 is a schematic diagram of an exploded structure of the suture control device in the embodiment of the present disclosure;
Figure 11 is a structural schematic diagram of a reel seat in the embodiment of the present disclosure;
Figure 12 is a schematic diagram of an assembly structure of the reel seat in the embodiment of the present disclosure;
Figure 13 is a schematic diagram of a sectional structure of the suture control device in a G-G direction in Figure 10;
Figure 14 is a structural schematic diagram of a thread return knob in the embodiment of the present disclosure.
Figure 15 is a schematic diagram of a sectional structure of a suture grasping device in the embodiment of the present disclosure;
Figure 16 is a structural schematic diagram of a catching net in a retracted state in the embodiment of the present disclosure;
Figure 17 is a structural schematic diagram of a double-layer catching net in an expanded state in the embodiment of the present disclosure;
Figure 18 is a schematic diagram of an application scenario of the suture grasping device in the embodiment of the present disclosure;
Figure 19 is a schematic diagram of an explosion structure of a bending adjustment device in the embodiment of the present disclosure;
Figure 20 is a schematic diagram of a sectional structure of the bending adjustment device in the embodiment of the present disclosure;
Figure 21 is a schematic diagram of a partial structure of the bending adjustment device in the embodiment of the present disclosure;
Figure 22 is a schematic diagram of an assembly structure of a front-end seat and a front end of a main tube in the embodiment of the present disclosure;
Figure 23 is a structural schematic diagram of a first rotating disk in the embodiment of the present disclosure;
Figure 24 is a structural schematic diagram of the front-end seat in the embodiment of the present disclosure;
Figure 25 is a schematic diagram of an assembly structure of the front-end seat in the embodiment of the present disclosure;
Figure 26 is a schematic diagram of an assembly structure of a first bending adjustment structure and a second bending adjustment structure in the embodiment of the present disclosure;
Figure 27 is a schematic diagram of a partial structure of the bending adjustment device in an embodiment of the present disclosure;
Figure 28 is a schematic diagram of an explosion structure of a first exhaust structure in the embodiment of the present disclosure;
Figure 29 is a schematic diagram of a sectional structure of the first exhaust structure in the embodiment of the present disclosure;
Figure 30 is a schematic diagram of an application scenario of the exhaust device in the embodiment of the present disclosure;
Figure 31 is a schematic diagram of a second exhaust structure in the embodiment of the present disclosure;
Figure 32 is a schematic diagram of a main tube and a sleeve pipe body structure in the embodiment of the present disclosure;
Figure 33 is a schematic diagram of an application scenario of the exhaust device in the embodiment of the present disclosure;
Figure 34 is a schematic diagram of an application scenario of the exhaust device in the embodiment of the present disclosure;
Figure 35 is a schematic diagram of an application scenario of the exhaust device in the embodiment of the present disclosure;
Figure 36 is a structural schematic diagram of the suture system in the embodiment of the present disclosure;
Figure 37 is a schematic diagram of an application scenario of the suture system in the embodiment of the present disclosure;
Figure 38 is a schematic diagram of a use scenario of the suture system in the embodiment of the present disclosure;
Figure 39 is a schematic diagram of a use scenario of the suture system in the embodiment of the present disclosure;
Figure 40 is a schematic diagram of a use scenario of the suture system in the embodiment of the present disclosure;
Figure 41 is a schematic diagram of a use scenario of the suture system in the embodiment of the present disclosure;
Figure 42 is a schematic diagram of a use scenario of the suture system in the embodiment of the present disclosure;
Figure 43 is a schematic diagram of a use scenario of the suture system in the embodiment of the present disclosure;
Figure 44 is a schematic diagram of a use scenario of the suture system in the embodiment of the present disclosure;
Figure 45 is a schematic diagram of a use scenario of the suture system in the embodiment of the present disclosure;
Figure 46 is a schematic diagram of a use scenario of the suture system in the embodiment of the present disclosure;
Figure 47 is a schematic diagram of a use scenario of the suture system in the embodiment of the present disclosure;
Figure 48 is a schematic diagram of a use scenario of the suture system in the embodiment of the present disclosure;
Figure 49 is a schematic diagram of a use scenario of the suture system in the embodiment of the present disclosure; and
Figure 50 is a schematic diagram of a use scenario of the suture system in the embodiment of the present disclosure.

Explanation of reference numerals: needle ejection device 1000: 1010. needle ejection handle, 1011. needle ejection top cover, 10111. locking hole, 10112. guide groove, 10113. adjustment hole, 10114. assembly hole, 10115. partition plate, 10116. partition position, 1012. needle ejection bottom cover, 10121. mother tooth portion, 1020. guide member, 1021. first baffle plate, 1022. second baffle plate, 1023. third baffle plate, 1024. conical block, 10241. puncture groove, 1025. splicing plate, 10251. convex plate, 1026. needle threading groove, 10261. first needle threading groove, 10262. second needle threading groove, 1027. locking groove, 1028. main body channel, 1030. suture needle, 1040. pushing body, 1041. first pushing body, 1042. second pushing body, 1043. pushing main body, 10431. head end, 10432. tail end, 1044. needle holder, 1050. locking member, 1051. locking post, 1052. locking plate, 1053. stop block, 1054. locking cap, 1060. locking strip, 1070. first push shaft, 1080. second push shaft, 1090. push rod, 1100. push button, 1110. pressing structure, 1111. pressing plate, 1112. elastic sleeve, 1113. clamping member, 1114. sub-tooth portion, 1115. connecting rod; suture control device 2000: 2010. suture, 2011. abutment head, 2020. suture pushing assembly, 2021. suture pushing wire, 2022. first tube body, 2023. second tube body, 2024. limiting joint, 2030. suture handle, 2031. suture top cover, 20311. thread return hole, 2032. suture bottom cover, 2033. adapter groove, 2040. adapter joint, 2041. first docking hole, 2042. second docking hole, 2043. conical hole, 2050. guide tube, 2051. trumpet tube, 2060. reel seat, 2061. reel hole, 2062. thread pushing groove, 2063. first seat body, 2064. second seat body, 2065. thread pushing hole, 2066. bearing hole, 2070. thread drum, 2071. marking position, 2080. thread return assembly, 2081. thread return shaft, 20811. retaining ring, 2082. bearing, 2083. thread return knob, 20831. buckle part, 20832. protrusion portion, 2084. nut, 2090. sleeve, 2091. limit ring, 2100. docking tube, 2101. buckle ring, 2110. protective cover; suture grasping device 3000: 3010. capture net, 3020. connecting tube, 3021. proximal end, 3022. distal end, 3023. developing ring, 3030. reinforcing member, 3040. support tube, 3050. state switching wire; bending adjustment device 4000: 4010. main tube, 4011. first chamber, 40111. first wire entry hole, 4012. second chamber, 4013. third chamber, 40131. needle threading hole, 4014. second wire entry hole, 4015. needle ejection section, 4016. first flexible section, 4017. second flexible section, 4018. main section, 4020. first bending adjustment structure, 4021. first rotating disk, 40211. first projection, 4022. first rotating shaft, 4023. first bending adjustment wire, 4024. first positioning bead, 4025. first wire connecting rod, 4026. first bending adjustment cap, 4030. fixed block, 4031. mounting channel, 4032. assembly plate, 4040. front-end seat, 4041. positioning rod, 4042. wire threading hole, 4043. axial hole, 4050. guide post, 4051. guide hole, 4052. developing part, 4060. adapter post, 4070. second bending adjustment structure, 4071. second rotating shaft, 4072. second rotating disk, 4073. second bending adjustment wire, 4074. second wire connecting rod, 4075. second bending adjustment cap, 4080. adapter seat, 4090. bending adjustment handle, 4091. bending adjustment top cover, 4092. bending adjustment bottom cover; exhaust device 5000: 5010. first exhaust structure, 5011. exhaust pipe, 5012. first knob, 5013. screw cap, 5 014. first sealing ring, 5015. sealing ring, 5016. receiving tube, 5011a. first channel, 5011b. second channel, 5011c. liquid injection tube, 5011d. liquid injection channel, 5020. second exhaust structure, 5021. first tubular object, 5022. second knob, 5023. second tubular object, 5024. valve, 5030. inner tube, 5040. reinforced sheath core, 5050. sheath tube, 5060. guide wire; A. foramen ovale, A1. atrial septum, A2. right atrium, A3. left atrium, B. first visual window, C. second visual window.

### DETAILED DESCRIPTION OF EMBODIMENTS

The present disclosure is further described in detail below by specific embodiments in combination with the drawings. Similar elements in different embodiments are associated with similar element numbers. In the following embodiments, many detailed descriptions are intended to make the present disclosure better understood. However, those skilled in the art can easily recognize that some of the features can be omitted in different situations, or can be replaced by other elements, materials, and methods. In some cases, some operations related to the present disclosure are not shown or described in the specification. This is to avoid the core part of the present disclosure being overwhelmed by too much description. For those skilled in the art, it is not necessary to describe these related operations in detail. They can fully understand the related operations based on the description in the specification and the general technical knowledge in the field.

In addition, the features, operations or characteristics described in the specification can be combined in any appropriate manner to form various embodiments. At the same time, the steps or actions in the method description can also be interchanged or adjusted regarding the orders in a manner that is obvious to those skilled in the art. Therefore, the various orders in the specification and the drawings are only for the purpose of clearly describing an embodiment, and do not mean that they are necessary orders, unless otherwise specified that an order must be followed.

The serial numbers themselves, such as "first", "second", etc., assigned to the components herein are only configured to distinguish the objects described and do not have any order or technical meaning. The "connection" and "coupling" mentioned in the present disclosure include direct and indirect connection (coupling) unless otherwise specified.

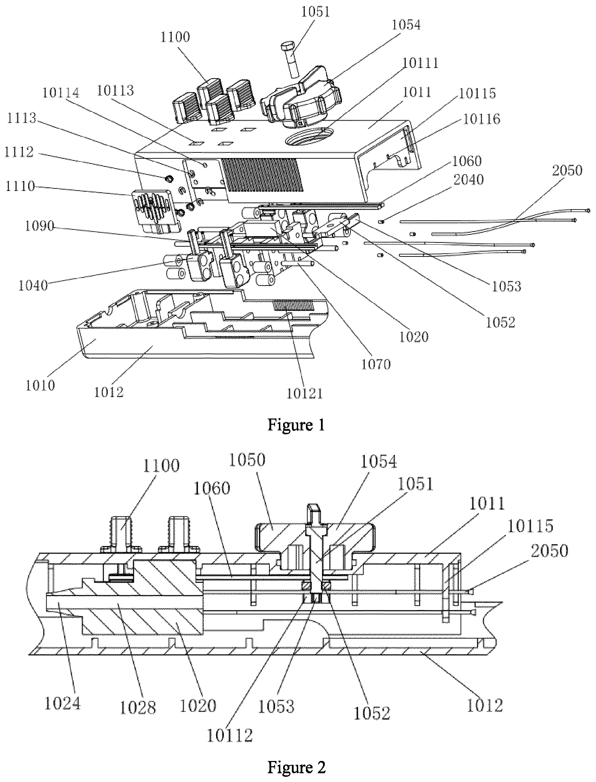

As shown in Figure 1 to Figure 14, a suture system is provided in an embodiment of the present disclosure. The suture system comprises a needle ejection device 1000 and a suture control device 2000. The needle ejection device 1000 is configured to push out or retract the suture needle 1030, and the suture control device 2000 is configured to push or retract the suture 2010.

The needle ejection device 1000 includes a needle ejection handle 1010, a guide member 1020, a pushing body 1040 and a suture needle 1030. The needle ejection handle 1010 includes a needle ejection top cover 1011 and a needle ejection bottom cover 1012, which are slidably connected. The guide member 1020 is located in the needle ejection handle 1010. The guide member 1020 and the needle ejection top cover 1011 are fixedly connected. The pushing body 1040 and the guide member 1020 are movably connected.

At least two suture needles 1030 are configured, and the suture needles 1030 are fixed to the pushing body 1040. The pushing body 1040 and the suture needles 1030 are arranged in a one-to-one correspondence. For example, two suture needles, three suture needles, four suture needles, etc. can be provided. In the embodiment of the present disclosure, four suture needles 1030 are used as an example to illustrate the structural design. The number of suture needles 1030 is flexibly selected according to the situation, and the embodiment of the present disclosure does not impose specific restrictions to it.

The needle ejection top cover 1011 is configured to slide relative to the needle ejection bottom cover 1012 to drive the guide member 1020 to move. The pushing body 1040 is configured to move along the guide member 1020 to drive the connected suture needles 1030 to move.

Using the suture system in the above embodiment of the present disclosure, when suturing the foramen ovale A, the needle ejection top cover 1011 can slide relative to the needle ejection bottom cover 1012 to synchronously drive the guide member 1020 and the multiple suture needles 1030 on the guide member 1020 to move synchronously. When a suture needle 1030 is not in a suitable position, the pushing body 1040 connected to the suture needle 1030 can be used to adjust the unsuitable position of the needle without re-puncturing wholly. That is, the needle ejection device 1000 in the designed suture system can realize both synchronous needle ejection and individual needle ejection, effectively improving the use efficiency and safety of the suture system, improving the puncture effect of the suture needle 1030, and improving the success rate of the operation.

As shown in Figure 1 and Figure 2, the needle ejection device 1000 also includes a locking member 1050 and at least two locking strips 1060. The locking strips 1060 are fixed to the pushing body 1040. The locking member 1050 has a first state and a second state. In the first state, the locking member 1050 is configured to lock at least two locking strips 1060 at the same time. In the second state, the locking member 1050 is configured to release the locking of at least two locking strips 1060.

By the cooperation of the locking member 1050 and the locking strip 1060, when the needle ejection device 1000 moves synchronously, the multiple suture needles 1030 on the guide member 1020 can move synchronously, so as to prevent a needle from moving or shaking alone and interfering with the consistency of the simultaneous pushing out or retracting of multiple suture needles 1030. When it is necessary to move a suture needle 1030 alone, the locking member 1050 is placed in the second state to facilitate the individual movement of a suture needle 1030.

As shown in Figure 1 and Figure 2, specifically, the locking member 1050 includes a locking post 1051 and a locking plate 1052. A locking hole 10111 is provided on the needle ejection top cover 1011, and the locking plate 1052 is located in the needle ejection handle 1010. A portion of the locking post 1051 passes through the locking hole 10111 and is threadedly connected to the locking plate 1052, and the remaining portion of the locking post 1051 is placed outside the needle ejection handle 1010. In the first state, the locking plate 1052 abuts against at least two locking strips 1060. In the second state, the locking plate 1052 and at least two locking strips 1060 are separated. The locking post 1051 is configured to change the position of the locking plate 1052 relative to the at least two locking strips 1060 by rotation.

As shown in Figure 1 and Figure 2, the locking member 1050 also includes a stop block 1053 and a locking cap 1054. The two side walls on the inner side of the needle ejection top cover 1011 are respectively provided with guide grooves 10112. The two ends of the locking plate 1052 are respectively slidably connected to the guide grooves 10112. When the locking post 1051 is rotated, the locking plate 1052 slides relative to the guide grooves 10112 to change the position of the locking plate 1052 relative to the locking strips 1060. The locking cap 1054 is fixedly connected to the locking post 1051 outside the needle ejection handle 1010. A stop block 1053 is fixed at one end of the guide groove 10112 away from the locking cap 1054. The stop block 1053 limits the movement range of the locking plate 1052 to prevent the locking plate 1052 from being separated from the guide groove 10112, thereby preventing the locking plate 1052 from being separated from the locking post 1051.

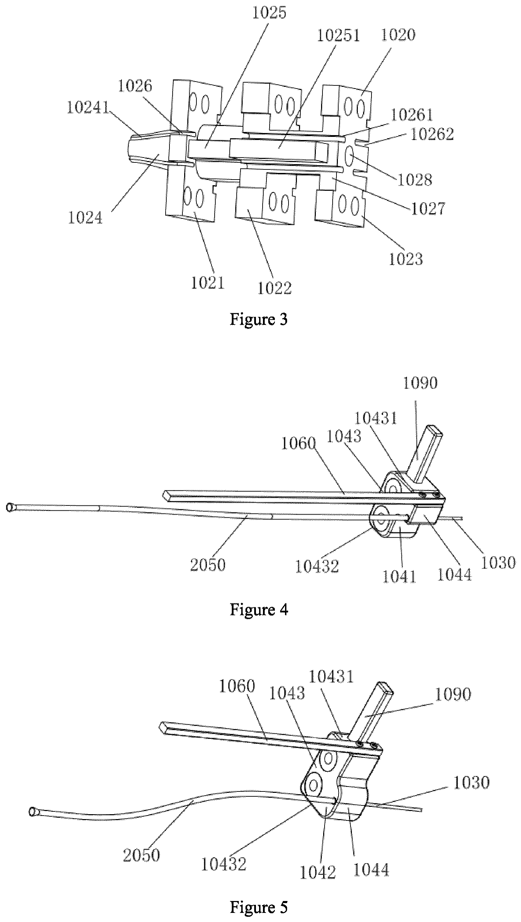

As shown in Figure 3, the guide member 1020 includes a first baffle 1021, a second baffle 1022, a third baffle 1023, and a conical block 1024. The conical block 1024 is fixed to the side of the first baffle 1021 away from the second baffle 1022. The pushing body 1040 includes a first pushing body 1041 and a second pushing body 1042, and two first pushing bodies 1041 and two second pushing bodies 1042 are configured. Two suture needle 1030 are fixed on the same side of the first pushing body 1041 and the second pushing body 1042 close to the conical block 1024. Four puncture grooves 10241 are provided on the outer wall of the conical block 1024, and the four puncture grooves 10241 are arranged in an array around the axis of the conical block 1024. One end of the suture needle 1030 away from the third baffle 1023 penetrates into the puncture groove 10241, and the conical block 1024 is configured to guide the suture needles 1030 into the main tube 4010. A locking strip 1060 is fixed on each of the first pushing body 1041 and the second pushing body 1042. A first push shaft 1070 is fixed between the first baffle 1021 and the second baffle 1022, and the first pushing body 1041 is movably sleeved on the first push shaft 1070. At least one first push shaft 1070 is sleeved on each first pushing body 1041. The second push shaft 1080 is fixed between the second baffle 1022 and the third baffle 1023, and the second pushing body 1042 is movably sleeved on the second push shaft 1080. At least one second push shaft 1080 is sleeved on each second pushing body 1042. A plurality of adjustment holes 10113 are provided on the needle ejection top cover 1011. Push rods 1090 are fixed on the sides of the first pushing body 1041 and the second pushing body 1042 facing the needle ejection top cover 1011. Each of the pushing rods 1090 passes through one of the adjustment holes 10113 and is placed outside the needle ejection handle 1010. A push button 1100 is sleeved on the push rod 1090 outside the needle ejection handle 1010, and the push button 1100 facilitates the application of force to push the push rod 1090.

As shown in Figure 4 and Figure 5, each first pushing body 1041 includes a pushing main body 1043 and a needle holder 1044 connected to each other. Each pushing main body 1043 has a head end 10431 and a tail end 10432. The head end 10431 is located the side near the needle ejection top cover 1011, and the tail end 10432 is located the side near the needle ejection bottom cover 1012. Two first push shafts 1070 are movably sleeved by the pushing main body 1043 on the first pushing body 1041, and two second push shafts 1080 are movably sleeved by the pushing main body 1043 on the second pushing body 1042. The suture needles 1030 on the first pushing body 1041 and the second pushing body 1042 are fixed on the needle holder 1044.

The needle holder 1044 in the first pushing body 1041 is fixed to the side wall of the pushing main body 1043. The surfaces of the pushing main body 1043 and the needle holder 1044 in the first pushing body 1041 close to the needle ejection top cover 1011 are flush, and the needle holders 1044 are fixed on the sides of the pushing main bodies 1043 in the two first pushing bodies 1041 close to each other. The needle holder 1044 in the second pushing body 1042 is fixed to the side wall of the pushing main body 1043, and the surfaces of the pushing main body 1043 and the needle holder 1044 in the second pushing body 1042 close to the needle ejection bottom cover 1012 are flush, and the needle holders 1044 are fixed on the sides of the pushing main bodies 1043 in the two second pushing bodies 1042 close to each other. The locking strip 1060 on the first pushing body 1041 is fixed to the side of the needle holder 1044 facing the needle ejection top cover 1011. The locking strip 1060 on the second pushing body 1042 is fixed on the side of the pushing main body 1043 facing the needle ejection top cover 1011.

As shown in Figure 3, the guide member 1020 also includes a splicing plate 1025, and the splicing plate 1025 is connected to the first baffle 1021, the second baffle 1022 and the third baffle 1023, and is perpendicular to the first baffle 1021, the second baffle 1022 and the third baffle 1023. The first baffle 1021 is provided with a convex plate 10251 on the side facing the needle ejection top cover 1011, and the convex plate 10251 and the needle ejection top cover 1011 are fixedly connected.

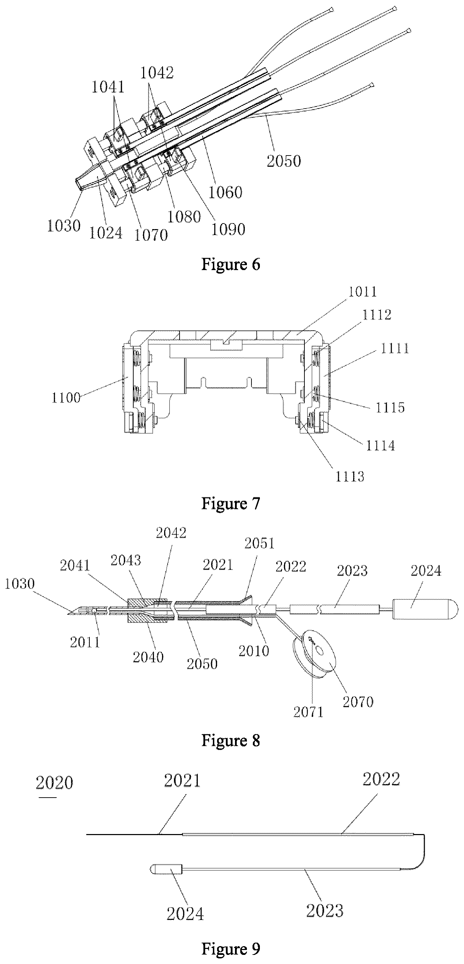

As shown in Figure 6, the first pushing body 1041 and the second pushing body 1042 are mounted on the guide member 1020, and the suture needle 1030 and the locking strip 1060 are mounted on the first pushing body 1041 and the second pushing body 1042 respectively. One end of each of the four locking strips 1060 is fixed to the corresponding pushing body 1040, and the other end of each of the four locking strips 1060 extends away from the end of the conical block 1024, respectively. The four locking strips 1060 are arranged side by side, so that the four locking strips 1060 can be locked by a locking plate 1052. The two first pushing bodies 1041 and the two second pushing bodies 1042 are all symmetrically distributed about the axis of the conical block 1024. Combined with the respective shape designs of the first pushing body 1041 and the second pushing body 1042 and the distribution of the suture needles 1030 thereon, the axes of the four suture needles 1030 can form a standard rectangular body. The axes of the four suture needles 1030 and the axes of the four puncture grooves 10241 on the conical block 1024 also correspond one to one, so that the four suture needles 1030 can accurately penetrate into the corresponding puncture grooves 10241.

As shown in Figure 3, the guide member 1020 is provided with a needle threading groove 1026 corresponding to the number of the suture needles 1030, and the needle threading grooves 1026 are specifically divided into a first needle threading groove 10261 and a second needle threading groove 10262. The guide member 1020 is provided with two first needle threading grooves 10261 arranged side by side on the side facing the needle ejection top cover 1011, and the first needle threading groove 10261 is configured to allow the suture needles 1030 on the first pushing body 1041 to pass through. The guide member 1020 is provided with two second needle threading grooves 10262 arranged side by side on the side facing the needle ejection bottom cover 1012, and the second needle threading grooves 10262 are configured to allow the suture needles 1030 on the second pushing body 1042 to pass through. The first needle threading groove 10261 and the second needle threading groove 10262 penetrate the first baffle 1021, the second baffle 1022 and the third baffle 1023. The guide member 1020 has two locking grooves 1027 on the side facing the needle ejection top cover 1011. The locking grooves 1027 are configured to allow the locking strips 1060 to pass through. The two locking grooves 1027 are symmetrically distributed about the axis of the conical block 1024. The locking grooves 1027 pass through the second baffle 1022 and the third baffle 1023. The guide member 1020 also has a main body channel 1028, and the main body channel 1028 is configured to allow the main tube 4010 to pass through. The main body channel 1028 passes through the first baffle 1021, the second baffle 1022, the third baffle 1023 and the conical block 1024.

As shown in Figure 7, pressing structures 1110 are symmetrically arranged on the side walls of the needle ejection top cover 1011. The pressing structure 1110 includes a pressing plate 1111, an elastic sleeve 1112 and a clamping member 1113. Sub-tooth portions 1114 and connecting rods 1115 are respectively arranged on both sides of the pressing plate 1111. The sub-tooth portions 1114 are located on the side away from the needle ejection top cover 1011, and the connecting rods 1115 are located on the side close to the needle ejection top cover 1011. Multiple connecting rods 1115 are configured. For example, four connecting rods 1115 are configured. One end of each connecting rod 1115 away from the pressing plate 1111 passes through the side wall of the needle ejection top cover 1011 and is placed in the needle ejection top cover 1011. The side wall of the needle ejection top cover 1011 is provided with an assembly hole 10114 corresponding to each connecting rod 1115, so that the connecting rods 1115 can pass through the side wall of the needle ejection top cover 1011. The elastic sleeve 1112 is arranged on each connecting rod 1115 between the pressing plate 1111 and the needle ejection top cover 1011. The clamping member 1113 is fixed to the end of each connecting rod 1115 away from the pressing plate 1111. The elastic sleeve 1112, the clamping member 1113 and the connecting rod 1115 are arranged in a one-to-one correspondence.

The mother tooth portions 10121 are symmetrically arranged on the side walls of the needle ejection bottom cover 1012, and the sub-tooth portions 1114 are configured to mesh with the mother tooth portions 10121. The number of teeth of the sub-tooth portion 1114 is less than the number of teeth of the mother tooth portion 10121. The elastic sleeve 1112 is, for example, a conical spring, and the clamping member 1113 is, for example, a pin, a retaining spring, etc. Through the pressing structure 1110 and the second tooth portion, it is convenient to quickly realize the locking or unlocking between the needle ejection top cover 1011 and the needle ejection bottom cover 1012, saving the use time of the instrument.

As shown in Figure 8, the suture control device 2000 includes a suture 2010 and a suture pushing assembly 2020. The end of the suture 2010 is configured to penetrate into the suture needle 1030, and the suture 2010 located in the suture needle 1030 is provided with an abutment head 2011. The suture pushing assembly 2020 includes a suture pushing wire 2021, one end of the suture pushing wire 2021 abuts against the abutment head 2011, and the other end of the suture pushing wire 2021 is located outside the needle ejection handle 1010. The suture pushing wire 2021 is configured to push the suture 2010. The abutment head 2011 can be produced, for example, by tying a knot in the suture 2010, or the abutment head 2011 is a ring body with a developing function. When the thread needs to be pushed, the thread pushing function can be quickly realized by cooperating with the abutment head 2011 and the suture pushing wire 2021 on the suture 2010.

It should be noted that, in the embodiment of the present disclosure, the suture needle 1030 can accommodate a suture 2010 and a suture pushing wire 2021, but cannot accommodate a suture pushing wire 2021 and an abutment head 2011. That is, the suture pushing wire 2021 cannot be placed side by side with the abutment head 2011 in the suture needle 1030. The suture pushing wire 2021 and the abutment head 2011 need to be staggered in the suture needle 1030, so that it is also convenient to ensure the abutment between the suture pushing wire 2021 and the abutment head 2011.

As shown in Figure 8 and Figure 9, the suture pushing assembly 2020 also includes a first tube body 2022, a second tube body 2023 and a limit joint 2024. The first tube body 2022 is fixedly sleeved on the suture pushing wire 2021, and the second tube body 2023 is movably sleeved on the suture pushing wire 2021. One end of the suture pushing wire 2021 is the thread pushing end, and the other end of the suture pushing wire 2021 is the free end. The first tube body 2022 is located on a side near the thread pushing end, and the second tube body 2023 is located on a side near the free end. The limit joint 2024 is fixedly connected to the free end.

The function of the first tube body 2022 is to connect the suture pushing wire 2021 so that the external force can push the suture pushing wire 2021 forward when pushing the first tube body 2022. The function of the second tube body 2023 is to strengthen the pushing strength (because the suture pushing wire 2021 is relatively soft and inconvenient to push directly). The structures of the first tube body 2022 and the second tube body 2023 can be the same, and the radial dimension of the limit joint 2024 is larger than the radial dimension of the second tube body 2023 to prevent the second tube body 2023 and the suture pushing wire 2021 from separating. The designed suture pushing assembly 2020 ensures the stability of the push wire process and improves the push wire speed (compared with directly pushing the suture 2010 or pushing the suture pushing wire 2021 to drive the suture 2010 forward). The first tube body 2022 and the second tube body 2023 are made of metal, such as stainless steel, and the suture pushing wire 2021 is nickel-titanium wire.

As shown in Figure 8, Figure 10 and Figure12, the suture control device 2000 also includes a suture handle 2030, an adapter joint 2040 and a guide tube 2050. The suture handle 2030 is provided with an adapter groove 2033. The adapter joint 2040 is provided with a first docking hole 2041 and a second docking hole 2042 that are communicated in the axial direction. The suture needle 1030 is arranged at one end of the adapter joint 2040 and the guide tube 2050 is arranged at the other end of the adapter joint 2040. Part of the suture needle 1030 is fixedly sleeved in the first docking hole 2041, and part of the guide tube 2050 is fixedly sleeved in the second docking hole 2042. The outer diameter of the suture needle 1030 is smaller than the outer diameter of the guide tube 2050. In order to facilitate the suture 2010 to enter the suture needle 1030 from the guide tube 2050 and then perform the thread pushing operation, a conical hole 2043 is opened in the adapter joint 2040 between the first docking hole 2041 and the second docking hole 2042. From the side close to the second docking hole 2042 to the side away from the second docking hole 2042, the aperture of the conical hole 2043 is continuously reduced. A trumpet tube 2051 can also be designed at the end of the guide tube 2050 away from the adapter joint 2040 to facilitate the suture 2010 to enter the guide tube 2050.

As shown in Figure 4 and Figure 5, the adapter joint 2040 for fixing the suture needle 1030 and the guide tube 2050 is fixed in the corresponding hole or channel opened on the needle holder 1044. By the adapter joint 2040, it is convenient to realize the assembly of the suture needle 1030 and the guide tube 2050. As shown in Figure 1, the adapter joint 2040 and part of the guide tube 2050 are placed in the needle ejection handle 1010. The needle ejection handle 1010 is connected to the suture handle 2030, and the suture handle 2030 is located on the side away from the conical block 1024.

Part of the first tube body 2022 close to the thread pushing end can be movably placed in the guide tube 2050 to facilitate rapid thread-pushing. The second tube body 2023 and the limit joint 2024 are located outside the suture handle 2030, and the second tube body 2023 can be detachably fixed in the adapter groove 2033. The second tube body 2023 can be temporarily stored through the adapter groove 2033, and the adapter groove 2033 also has the function of preventing the second tube body 2023 from being lost. At the same time, by movably sleeved the second tube body 2023 on the suture pushing wire 2021, the axial dimension of the suture control device 2000 can be made not too long, which is conducive to the miniaturization of the device design.

The suture 2010, the suture pushing assembly 2020, the adapter joint 2040, the guide tube 2050 and the suture needle 1030 are arranged in a one-to-one correspondence. When the suture 2010 needs to be pushed out of the suture needle 1030, the second tube body 2023 penetrates into the suture handle 2030 and abuts against the first tube body 2022, and the thread pushing end abuts against the abutment head 2011.

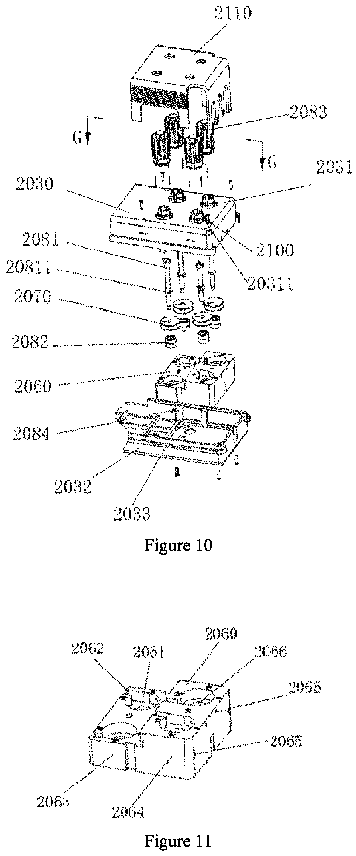

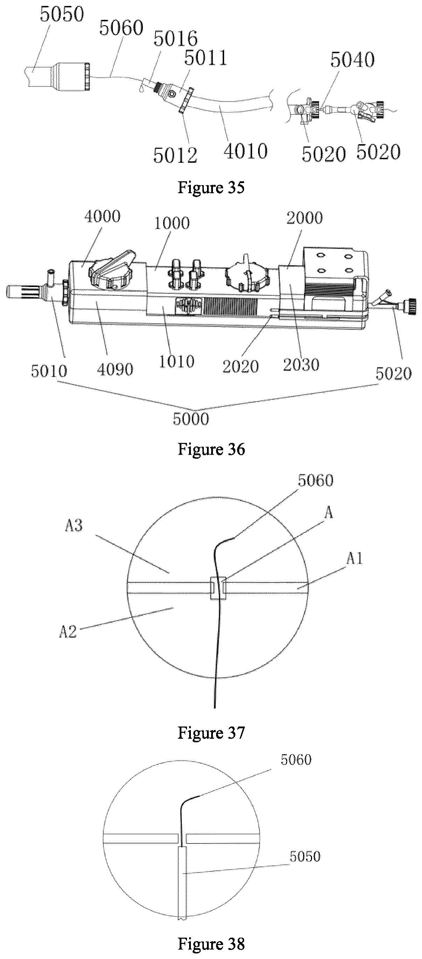

As shown in Figure 10 to Figure13, the suture handle 2030 includes a suture top cover 2031 and a suture bottom cover 2032, and the needle ejection bottom cover 1012 and the suture bottom cover 2032 can be an integrated structure. As shown in Figure 36, the suture handle 2030 is located on the right side of the needle ejection handle 1010. The suture control device 2000 also includes a reel seat 2060, a thread drum 2070 and a thread return shaft 2081 located in the suture handle 2030.

The reel seat 2060 is located at one end of the guide tube 2050 away from the adapter joint 2040. The reel seat 2060 is provided with a plurality of reel holes 2061 on the side facing the suture top cover 2031. The reel seat 2060 is provided with a plurality of thread pushing grooves 2062. The thread pushing grooves 2062 and the reel holes 2061 are arranged in a one-to-one correspondence. One end of the thread pushing groove 2062 is tangent to and communicated with the reel hole 2061. The other end of the thread pushing groove 2062 extends to the end surface of the reel seat 2060 close to the adapter joint 2040. A thread drum 2070 is rotatably fixed in the reel hole 2061. Multiple turns of suture 2010 are wound on the thread drum 2070. The end of the suture 2010 passes through the thread pushing groove 2062 and the guide tube 2050 and enters the suture needle 1030.

The thread return shaft 2081 is fixedly connected to the thread drum 2070, and the thread return shaft 2081 is configured to rotate the thread drum 2070 to perform a thread return operation. A corresponding thread return hole 20311 is provided on the suture top cover 2031, so that the thread return shaft 2081 can pass through and extend to the outside of the suture handle 2030. The suture top cover 2031 is made of transparent material, and a marking position 2071 is provided on the side of the thread drum 2070 facing the suture top cover 2031. In this way, if an abnormality occurs when pushing or catching the suture 210, it can be seen through the top cover that the marking position 2071 of the thread drum 2070 has not rotated, and the operation can be adjusted in time, and the abnormal suture 210 can be rewound through the thread return shaft 2081, and the thread can be pushed again through the suture pushing assembly 2020 after the thread is rewound. The thread drum 2070, the thread return shaft 2081, the reel hole 2061, the suture 2010, the suture pushing assembly 2020, the adapter joint 2040, the guide tube 2050 and the suture needle 1030 are all arranged in a one-to-one correspondence.

Multiple guide tubes 2050 are configured, and the multiple guide tubes 2050 are spaced apart in space. For example, four suture needle 1030 are configured, and four guide tubes 2050 are also correspondingly configured. As shown in Figure 1, a partition plate 10115 is provided at the end of the needle ejection top cover 1011 away from the conical block 1024, that is, the end close to the suture handle 2030, and four partition positions 10116 are provided on the partition plate 10115, and the partition positions 10116 are configured to allow the guide tube 2050 to pass through. The four partitions 10116 are grouped into two, one group of partitions 10116 is arranged side by side and located on the relatively upper side, the other group of partitions 10116 is also arranged side by side but located on the relatively lower side, and the group of partitions 10116 located on the lower side is arranged on both sides of the group of partitions 10116 located on the upper side. As shown in Figure 11, the reel seat 2060 includes a first seat body 2063 and a second seat body 2064, the first seat body 2063 is located on the side close to the suture needle 1030, and the second seat body 2064 is located on the side away from the suture needle 1030. The end surface of the first seat body 2063 facing the suture top cover 2031 is recessed downward relative to the end surface of the second seat body 2064 facing the suture top cover 2031, so as to form a stepped reel seat 2060. The end of the reel seat 2060 away from the suture needle 1030 is provided with four thread pushing holes 2065. The four thread pushing holes 2065 are divided into two groups, one group of thread pushing holes 2065 is arranged side by side and located on the relatively upper side, and the other group of thread pushing holes 2065 is arranged side by side and located on the relatively lower side. The group of thread pushing holes 2065 located on the lower side is distributed on both sides of the group of thread pushing holes 2065 located on the upper side, and the thread pushing holes 2065 and the thread pushing grooves 2062 correspond to each other and are communicated. The suture handle 2030 is provided with an operation hole (not shown) corresponding to the position of the thread pushing hole 2065. When the thread needs to be pushed, the second tube body 2023 is passed through the operation hole, the thread pushing hole 2065, the thread pushing groove 2062 and abuts against the first tube body 2022. Through the structural layout of the reel seat 2060 and the dispersed arrangement of the guide tube 2050 by the partition plate 10115, there is enough operating space when the four sutures 2010 are pushed, so as to prevent the four sutures 2010 from gathering and being inconvenient to push.

The suture control device 2000 also includes a thread return assembly 2080, and the thread return assembly 2080 includes a thread return shaft 2081, a bearing 2082, a thread return knob 2083 and a nut 2084. Corresponding bearing holes 2066 are opened on the first seat body 2063 and the second seat body 2064, and the bearing holes 2066 correspond to and are communicated to the reel hole 2061 one by one, and the bearing hole 2066 is located on the side of the reel hole 2061 away from the suture top cover 2031. A retaining ring 20811 is provided on the side wall of the thread return shaft 2081, and the thread drum 2070 is located between the retaining ring 20811 and the bearing 2082. The two ends of the thread drum 2070 are respectively in contact with the retaining ring 20811 and the bearing 2082. One end of the thread return shaft 2081 passes through the thread drum 2070 and the bearing 2082 and is threadedly connected with the nut 2084, and the other end of the thread return shaft 2081 is sleeved with the thread return knob 2083. The thread return shaft 2081 is driven to rotate by the thread return knob 2083, and the thread drum 2070 and the bearing 2082 are driven to rotate synchronously. The two ends of the inner ring of the bearing 2082 are respectively in contact with the thread drum 2070 and the nut 2084, so when the bearing 2082 rotates, it is the inner ring bearing 2082 that rotates, so that the thread drum 2070 has less resistance when rotating.

As shown in Figure 10 and Figure 13 to Figure 14, each thread return hole 20311 is provided with a sleeve 2090, the mouth of the sleeve 2090 is connected to the thread return hole 20311. One end of the docking tube 2100 is fixed to the bottom of the sleeve 2090, and the other end of the docking tube 2100 passes through the thread return hole 20311 and is placed on the outside of the suture handle 2030. A buckle ring 2101 is provided on the outer wall of the docking tube 2100, and a plurality of buckle parts 20831 are provided on the inner wall of the thread return knob 2083. For example, three buckle parts 20831 are provided, which are spaced apart along the axis of the thread return knob 2083. The middle buckle part 20831 is configured to be snapped with the buckle ring 2101, so that the thread return knob 2083 and the docking tube 2100 can be rotatably sleeved. The buckle parts 20831 on both sides can support the thread return knob 2083 to prevent the thread return knob 2083 from shaking relative to the docking tube 2100. The thread return shaft 2081 passes through the docking tube 2100 and sleeves the thread return knob 2083. A concave-convex matching structure is provided between the thread return shaft 2081 and the thread return knob 2083, so that the thread return knob 2083 and the thread return shaft 2081 can rotate synchronously.

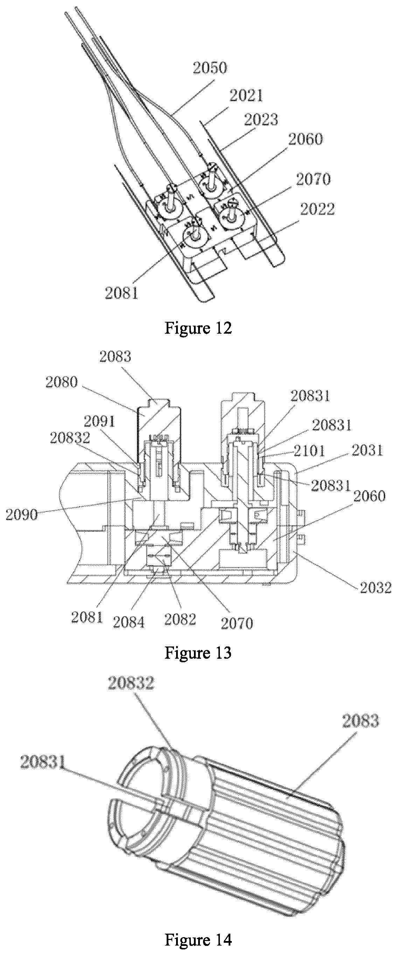

As shown in Figure 13, Figure 13 is a schematic diagram of the cross-sectional structure of the suture control device in Figure 10 along the G-G direction. In Figure 13, the protective cover 2110 in Figure 10 is omitted to clearly show the thread return knob 2083. A limit ring 2091 is provided on the side of the inner wall of the sleeve 2090 near the thread return hole 20311. A protrusion portion 20832 is provided on the outer wall of the thread return knob 2083, and the protrusion portion 20832 is located in the sleeve 2090. When the thread return shaft 2081 needs to be rotated, as shown in the thread return knob 2083 on the left side of Figure 13, the buckle part 20831 is snapped with the buckle ring 2101. The thread return knob 2083 and the end of the thread return shaft 2081 are concave-convex matched, and the protrusion portion 20832 and the buckle ring 2101 are separated. When the thread return shaft 2081 does not need to be rotated, the thread return knob 2083 is lifted upward, as shown in the right thread return knob 2083 in Figure 13, the buckle portion 20831 and the buckle ring 2101 are in a non-connected state. The thread return knob 2083 and the end of the thread return shaft 2081 are also released from the concave-convex matching relationship. But at this time, the protrusion portion 20832 and the limit ring 2091 are in contact, and the limit ring 2091 limits part of the thread return knob 2083 in the sleeve 2090, so that the thread return knob 2083 can move axially within a range.

As shown in Figure 10, the suture control device 2000 further includes a protective cover 2110, and the protective cover 2110 is detachably fixed on the top of the suture top cover 2031. The protective cover 2110 can cover multiple thread return knobs 2083 and multiple operation holes on the suture handle 2030 to prevent the first tube body 2022 in the suture handle 2030 from running out. The top of the protective cover 2110 and the thread return knob 2083 can be provided with a concave-convex matching structure to prevent the thread return knob 2083 from rotating by mistake when not in use.

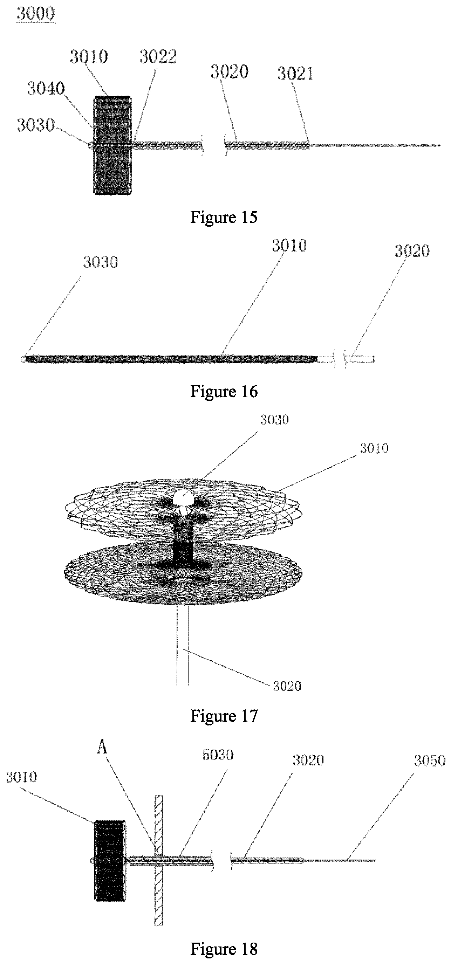

As shown in Figure 15 to Figure 18, the suture system further includes a suture grasping device 3000, wherein the suture grasping device 3000 includes: a capture net 3010, a connecting tube 3020, a reinforcing member 3030, a support tube 3040 and a state switching wire 3050.

The capture net 3010 has a contracted state and an expanded state. The capture net 3010 is strip-shaped in the contracted state and column-shaped in the expanded state. The strip-shaped capture net 3010 is convenient for storage and movement in the inner tube 5030. The columnar capture net 3010, such as the cylindrical capture net 3010, has a thickness space, which can prevent the suture needle 1030 from passing through the capture net 3010 and causing damage to other tissues around the foramen ovale A when the suture needle 1030 is inserted, and can also enhance the capture function of the suture 2010 to avoid the phenomenon of missing the suture 2010. As shown in Figure 17, the capture net 3010 can also be a double-layer net body, the relatively lower layer of the net body is configured for the suture needle 1030 to pass through, and the relatively upper layer of the net body prevents the suture needle 1030 from passing through and puncturing other human tissues.

The connecting tube 3020 has a proximal end 3021 and a distal end 3022. The distal end 3022 is connected to one end of the capture net 3010, and the proximal end 3021 can be located outside the suture handle 2030, so as to facilitate the application of force to push the connecting tube 3020 and the capture net 3010 forward in the inner tube 5030.

The reinforcing member 3030 is connected to the other end of the capture net 3010, and the reinforcing member 3030 is similar to a mortise or a ring. The reinforcing member 3030 is connected to one end of the capture net 3010, which can have a gathering effect on the capture wire or capture line at the end of the capture net 3010, so as to ensure the shape of the capture net 3010 and prevent the capture net 3010 from spreading.

The support tube 3040 is communicated with the connecting tube 3020. One end of the support tube 3040 is connected to the distal end 3022, the other end of the support tube 3040 is a free end. The support tube 3040 is located in the capture net 3010. The free end of the support tube 3040 is configured to abut against or sleeve on the reinforcing member 3030 on the capture net 3010 in the expanded state.

One end of the state switching wire 3050 passes through the connecting tube 3020 and the support tube 3040 into the capture net 3010 and is connected to the reinforcing member 3030, and the other end of the state switching wire 3050 is located outside the connecting tube 3020. The state switching wire 3050 is configured to move along the axial direction of the connecting tube 3020 to switch the state of the capture net 3010.

In the suture grasping device 3000 designed in the embodiment of the present disclosure, a strip-shaped capture net 3010 cooperates with the connecting tube 3020 to facilitate the movement and transportation of the capture net 3010 in the inner tube 5030, so that the capture net 3010 is completely placed outside the inner tube 5030. It should be noted here that when the suture system is used, the end of the inner tube 5030 will enter the human body and pass through the foramen ovale A to reach the left atrium A3. Correspondingly, when the capture net 3010 passes through the inner tube 5030 and is placed outside the inner tube 5030, the capture net 3010 is placed at the left atrium A3 outside the inner tube 5030. When the capture net 3010 is placed outside the inner tube 5030, the state switching wire 3050 is tightened, that is, the reinforcing member 3030 is pulled toward the side close to the connecting tube 3020, so that the state of the capture net 3010 changes from a strip to a columnar state, so as to facilitate the subsequent needle threading and pushing of the suture 2010. After pushing the suture 2010, the state switching wire 3050 moves in the opposite direction to switch the state of the capture net 3010 from columnar to strip, and then the capture net 3010 is withdrawn from the inner tube 5030 through the connecting tube 3020 to capture the suture 2010. The support tube 3040 is sleeved on or abutted against the reinforcing member 3030 when the capture net 3010 is in the expanded state, and plays a supporting role on the columnar capture net 3010, which is convenient for ensuring the thickness space of the capture net 3010, and then it is convenient for the capture net 3010 to prevent the suture needle 1030 from passing through and puncturing irrelevant human tissues, and it is also convenient for the capture net 3010 to better play the function of capturing the suture 2010.

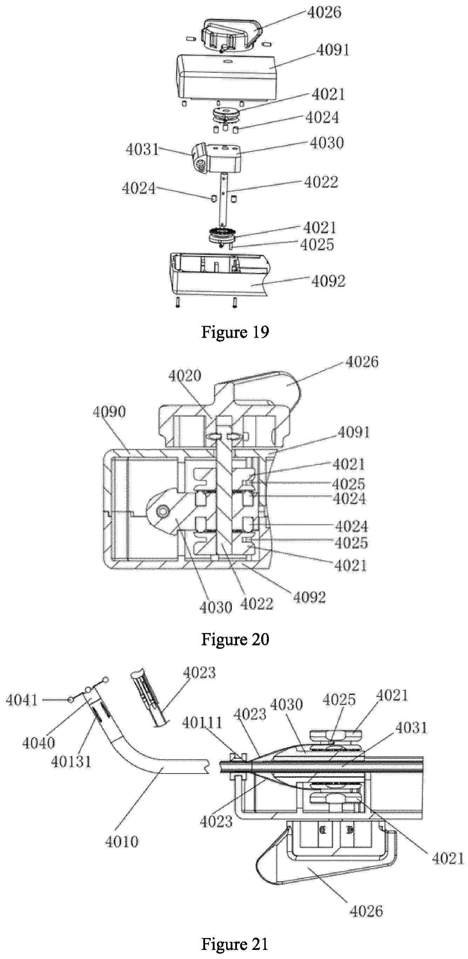

As shown in Figure 19 to Figure 27, the suture system also includes a bending adjustment device 4000, and the bending adjustment device 4000 includes a main tube 4010 and a first bending adjustment structure 4020. The main tube 4010 has a front end and a rear end. A first chamber 4011 is provided in the main tube 4010, and a first wire entry hole 40111 communicating with the first chamber 4011 is provided on the side wall of the main tube 4010 between the front end and the rear end. The first bending adjustment structure 4020 includes a first rotating disk 4021, a first rotating shaft 4022, and a first bending adjustment wire 4023. One end of the first bending adjustment wire 4023 is fixed to the front end of the main tube 4010, and the other end of the first bending adjustment wire 4023 passes through the first chamber 4011 and the first wire entry hole 40111 and is fixed to the first rotating disk 4021. The first rotating disk 4021 and the first rotating shaft 4022 are fixedly connected, and the first rotating disk 4021 is configured to rotate synchronously with the first rotating shaft 4022 to tighten or loosen the first bending adjustment wire 4023. By rotating the first rotating shaft 4022, the first rotating disk 4021 and the first bending adjustment wire 4023 can be driven to move synchronously, so as to adjust the bending of the segment of the main tube 4010 near the front end, ensure the puncture accuracy of the suture needle 1030, and improve the success rate of the operation.

As shown in Figure 19 to Figure 21 and Figure 23, the bending adjustment device 4000 also includes a fixed block 4030 and a first positioning bead 4024. The first rotating shaft 4022 is rotatably connected to the fixed block 4030, and two first rotating disks 4021 are configured. The two first rotating disks 4021 are located at both ends of the fixed block 4030. A plurality of first projections 40211 are provided on one end surface of the first rotating disk 4021 facing the fixed block 4030. The plurality of first projections 40211 are arranged in an array around the axis of the first rotating disk 4021, and a first clamping space is formed between adjacent first projections 40211. A plurality of first positioning beads 4024 are fixed to the fixed block 4030 on one side facing the first rotating disk 4021. The beads of the first positioning beads 4024 are clamped in the first clamping space. When the first rotating shaft 4022 rotates, the beads of the first positioning beads 4024 can be placed in different first clamping spaces. The first rotating disk 4021, the first bending adjustment wire 4023 and the first wire entry hole 40111 are all arranged in a one-to-one correspondence. The ends of the two first bending adjustment wires 4023 away from the front end of the main tube 4010 are distributed on both sides of the axis of the main tube 4010, so that when the first rotating shaft 4022 rotates, any one of the two first bending adjustment wires 4023 is in a tightened state, and the other of the two first bending adjustment wires 4023 is in a loosened state.

Two first rotating disks 4021 are provided, which are respectively arranged at both ends of the fixed block 4030. Combined with the position layout of the two first bending adjustment wires 4023, the designed bending adjustment device 4000 has positive and negative bending adjustment functions. For example, as shown in Figure 21, operating the first rotating shaft 4022 can make the main tube 4010 at the front end bend upward, or rotating the first rotating shaft 4022 in the opposite direction can make the main tube 4010 at the front end bend downward, so that the bending adjustment device 4000 has more usage scenarios or a larger range of usage. The first projection 40211 is provided with a curved surface so that the first positioning bead 4024 is tangent to and pass over the first projection 40211. The first projection 40211 and the first positioning bead 4024 are provided between each first rotating disk 4021 and the fixed block 4030, so that when the first rotating shaft 4022 is rotated, the first positioning bead 4024 can switch different first clamping spaces, and when the first rotating shaft 4022 stops rotating, the first positioning bead 4024 is clamped in the current first clamping space, that is, it has the function of stopping at any time. The first projection 40211 and the first positioning bead 4024 cooperate to achieve a self-locking function. The designed bending adjustment device 4000 can firmly ensure the current state of the bending adjustment device 4000 through the cooperation of the first projection 40211 and the first positioning bead 4024 when the first bending adjustment wire 4023 is subjected to a large tensile force, and the first bending adjustment wire 4023 is not easy to be unstable or shaken.

As shown in Figure 19 to Figure 21, a groove is provided on the side wall of the first rotating disk 4021 to facilitate winding of the first bending adjustment wire 4023. A first wire connecting rod 4025 is provided in the groove of the first rotating disk 4021. One end of the first bending adjustment wire 4023 away from the front end of the main tube 4010 is fixed to the first wire connecting rod 4025. The first wire connecting rod 4025 ensures that the first bending adjustment wire 4023 moves synchronously when the first rotating disk 4021 rotates. The bending adjustment device 4000 also includes a bending adjustment handle 4090, and the adjustment handle 4090 includes a bending adjustment top cover 4091 and a bending bottom cover 4092, which are connected to each other. As shown in Figure 36, the bending adjustment handle 4090 is located on the left side of the needle ejection handle 1010, and the bending adjustment bottom cover 4092 and the needle ejection bottom cover 1012 can be an integrated structure. The first bending adjustment structure 4020 also includes a first bending adjustment cap 4026. The first bending adjustment structure 4020 and the fixed block 4030 are assembled in the bending adjustment handle 4090. The first rotating shaft 4022 passes through the bending adjustment top cover 4091 and is fixedly connected to the first bending adjustment cap 4026. The fixed block 4030 is provided with a mounting channel 4031. The mounting channel 4031 is adapted to the shape of the outer wall of the main tube 4010. The mounting channel 4031 is configured to fix part of the main tube 4010.

As shown in Figure 21 to Figure 22 and Figure 24 to Figure 27, the bending adjustment device 4000 also includes a front-end seat 4040, and the front-end seat 4040 is connected to the front end of the main tube 4010. A wire threading hole 4042 is provided on the front-end seat 4040, and the first bending adjustment wire 4023 passes through the wire threading hole 4042 to become a double-strand bending adjustment wire. One end of the double-strand bending adjustment wire is fixed to the wire threading hole 4042, and the other end of the double-strand bending adjustment wire passes through the first chamber 4011 and is fixed to the first rotating disk 4021. Alternatively, the wire threading hole 4042 is not provided on the front-end seat 4040, but the bending adjustment device 4000 also includes an adapter post 4060, one end of which is fixed to one end of the front-end seat 4040 close to the main tube 4010, and the adapter post 4060 is fixedly connected to the first bending adjustment wire 4023. The adapter post 4060 is sleeved and fixed in the first chamber 4011. The front-end seat 4040 and the adapter seat 4080 are made of metal to ensure the fixing strength, for example, the front-end seat 4040 and the adapter seat 4080 are made of stainless steel.

A plurality of circumferentially equally spaced positioning rods 4041 are provided at one end of the front-end seat 4040 away from the main tube 4010, and the positioning rods 4041 are elastic. The number of the positioning rods 4041 is, for example, three or four. The positioning rods 4041 have two states, a retracted state and a spread state. When the front-end seat 4040 is placed in the sheath tube 5050 along with the main tube 4010, the plurality of positioning rods 4041 are in the retracted state, and when the front-end seat 4040 is placed in the right atrium A2 in the human body through the sheath tube 5050, the plurality of positioning rods 4041 are in a spread state due to their own elasticity. The multiple positioning rods 4041 in the spread state can facilitate the doctor to determine whether the front end of the suture device is in contact with the atrial septum A1, so as to perform subsequent surgical operations. At the same time, the positioning rods 4041 in the spread state can also play a supporting role in contacting the tissue around the foramen ovale A, and the capture net 3010 located in the left atrium A3 can also play a supporting role in contacting the tissue around the foramen ovale A. The multiple positioning rods 4041 and the columnar capture net 3010 clamp the tissue around the foramen ovale A on both sides, providing sufficient support effect for the tissue, and facilitating puncture.

The main tube 4010 also has a second chamber 4012 and multiple third chambers 4013. The second chamber 4012 is configured to allow the inner tube 5030 to pass through, and the third chamber 4013 is configured to allow the suture needles 1030 to pass through. The center of the front-end seat 4040 also has an axial hole 4043, and the axial hole 4043 and the second chamber 4012 are coaxially distributed, so that the inner tube 5030 can pass through and enter the left atrium A3. A needle threading hole 40131 is provided on the side wall of the main tube 4010 outside the third chamber 4013. The bending adjustment device 4000 further includes a plurality of guide posts 4050. One end of the guide post 4050 is fixed to one end of the front-end seat 4040 close to the main tube 4010. The other end of the guide post 4050 is placed in the third chamber 4013. The guide posts 4050 are configured to allow the suture needles 1030 to pass through the main tube 4010. A guide channel is provided in the guide posts 4050. A guide hole 4051 corresponding to the position of the needle threading hole 40131 is provided on the side wall of the guide posts 4050. The guide hole 4051 is communicated to one end of the guide channel. The other end of the guide channel is configured to allow the suture needle 1030 to enter. The guide posts 4050 guide the suture needles 1030 in the third chamber 4013, so that the suture needle 1030 can pass through the needle threading hole 40131 at a specific angle. At least two groups of developing parts 4052 are provided on the plurality of guide posts 4050. The number of developing parts in different groups can be the same or different. For example, two groups of developing parts 4052 are provided on the plurality of guide posts 4050. The number of developing parts 4052 in one group is one, and the number of developing parts 4052 in the other group is two. The developing parts 4052 are configured to distinguish the guide posts 4050 at different positions.

The plurality of third chambers 4013 are arranged in an array around the axis of the main tube 4010. Four third chambers 4013 are configured. The third chambers 4013, the guide posts 4050 and the suture needles 1030 are arranged in a one-to-one correspondence. Developing parts 4052 are respectively arranged on the outer side wall of any two adjacent guide posts 4050 among the four guide posts 4050. Any one of the two guide posts 4050 with developing parts 4052 has one developing part 4052 thereon, and the other of the two guide posts 4050 with developing parts 4052 has two developing parts 4052 thereon. A developing distance is formed between the two developing parts 4052 on the same guide post 4050, and the developing distance is greater than the axial length of the developing part 4052. At least one first bending adjustment wire 4023 is arranged between the two guide posts 4050 with developing parts 4052.

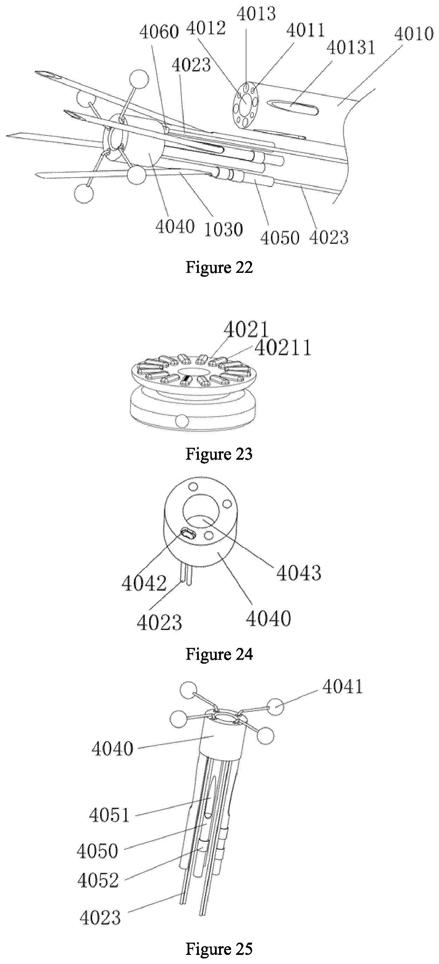

As shown in Figure 25, developing parts 4052 are arranged on the outer walls of some of the four guide posts 4050. For example, in the clockwise direction, there are one, two, three, or four guide posts 4050. If there is only one bending adjustment direction, the first bending adjustment wire 4023 can be arranged between the first guide post 4050 and the fourth guide post 4050, that is, one developing part 4052 is arranged on the first guide post 4050, and two developing parts 4052 are arranged on the fourth guide post 4050. The developing spacing formed between the two developing parts 4052 on the fourth guide post 4050 is not less than the axial length of the developing part 4052 on the first guide post 4050. This arrangement is convenient for doctors to observe that when the developing parts on the first guide post 4050 and the fourth guide post 4050 form three developing points on the developing device by rotating the developing device when it is in use. When the three developing points form a straight line, it is a suitable and safe needle ejection angle, and the doctor can then ejection the needle for subsequent surgery. If two directions of bending adjustment are required, two first bending adjustment wires 4023 are required to be respectively arranged between the first guide post 4050 and the fourth guide post 4050, and between the second guide post 4050 and the third guide post 4050. The developing parts 4052 can be respectively arranged on the first guide post 4050 and the fourth guide post 4050, or the developing parts 4052 can be respectively arranged on the second guide post 4050 and the third guide post 4050. It should be noted that the so-called developing point refers to the point-like state of the axial cross section of the developing part 4052 seen on the developing device. The actual cross section of the developing part 4052 is a rectangle. Since the cross section of the developing part 4052 is very small relative to the suture system, the developing part 4052 is regarded as a nearly point-like structure on the developing device.

The following description is based on the example of arranging the developing part 4052 on the first guide post 4050 and the fourth guide post 4050. For example, the developing parts 4052 are annular, the first guide post 4050 has one developing part 4052, and the fourth guide post 4050 has two developing parts 4052. Alternatively, the first guide post 4050 has a short developing part 4052 and the fourth guide post 4050 has a long developing part 4052. Alternatively, the first guide post 4050 has a developing pattern 1 engraved on it and the fourth guide post 4050 has a developing pattern 2 engraved on it. In short, the first guide post 4050 and the fourth guide post 4050 can be distinguished under X-ray, that is, the first suture needle 1030 and the second suture needle 1030 can be distinguished. At this time, in a clockwise direction, as shown in Figure 25, the order of the four guide posts 4050 can be distinguished. For example, the left side close to the first guide post 4050 is provided with the second guide post 4050. The four guide posts 4050 and the four push rods 1090 are pre-matched in a one-to-one correspondence.

The objective of providing the developing part 4052 on a specific guide post 4050 is to facilitate the one-to-one correspondence between the suture needle 1030 and its corresponding push rod 1090 when the device is exposed to X-rays, so as to facilitate the operation of the doctor and avoid the surgical risk caused by pushing the wrong suture needle 1030. When a suture needle 1030 needs to be adjusted individually, the developing part 4052 can help determine which push rod 1090 on the needle ejection handle 1010 corresponds to the suture needle 1030 that needs to be adjusted, thereby avoiding the risk of incorrectly adjusting the suture needle 1030.

Based on the function of the developing part 4052, the developing part 4052 can be replaced with other forms in addition to being ring-shaped. For example, cutting the guide post 4050 into a specific shape can also achieve a similar effect. The developing part 4052 can be not placed on the guide post 4050. It can be other precious metals such as a cylindrical one inlaid in any part of the front end of the main tube 4010, as long as it ensures a one-to-one correspondence with the suture needle 1030, and assists in determining the suture needle 1030. The number of the above-mentioned developing parts 4052 is only an example. The listed method can simplify the number of developing parts 4052 as much as possible, which helps to simplify the structure. Of course, in other embodiments, each guide post 4050 can be provided with a developing part 4052.

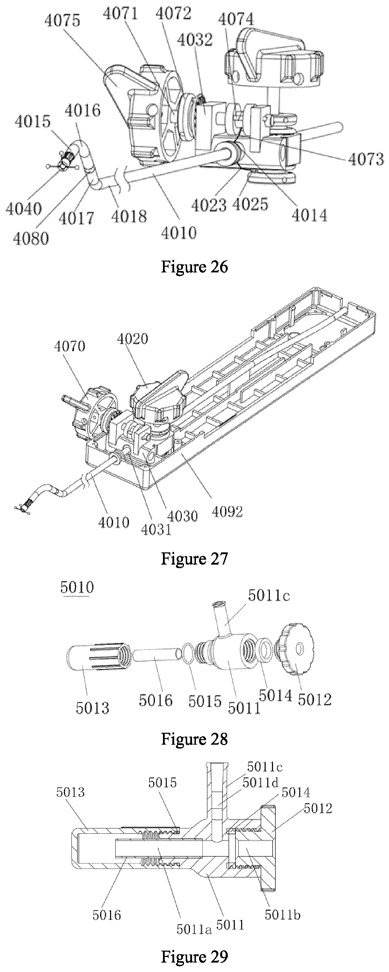

As shown in Figures 26 and 27, the bending adjustment device 4000 in the embodiment of the present disclosure also includes a second bending adjustment structure 4070. The second bending adjustment structure 4070 includes a second rotating shaft 4071, a second rotating disk 4072 and a second bending adjustment wire 4073. From the front-end side of the main tube 4010 to the rear-end side of the main tube 4010, the main tube 4010 includes a needle ejection section 4015, a first flexible section 4016, a second flexible section 4017 and a main section 4018 connected in sequence. The first bending adjustment structure 4020 is configured to bend the first flexible section 4016, the second bending adjustment structure 4070 is configured to bend the second flexible section 4017, and the bending direction of the second bending adjustment structure 4070 is opposite to that of the first bending adjustment structure 4020. For example, as shown in Figure 27, the first flexible section 4016 bends downward, and the second flexible section 4017 bends upward accordingly. By bending the second flexible section 4017, the position deviation generated by the bending of the first flexible section 4016 can be compensated.

An adapter seat 4080 is fixed between the first flexible section 4016 and the second flexible section 4017. A fourth chamber (not shown) is provided in the main tube 4010, and a second wire entry hole 4014 communicating with the fourth chamber is provided on the side wall of the main tube 4010 between the front end and the rear end. According to the use scenario of the second bending adjustment wire 4073, the fourth chamber can be provided on the main tube 4010 between the adapter seat 4080 and the second wire entry hole 4014. Similarly, according to the use scenario of the first bending adjustment wire 4023, the first chamber 4011 can be provided on the main tube 4010 between the front end of the main tube 4010 and the first wire entry hole 40111.

The second rotating shaft 4071 is fixedly connected to the second rotating disk 4072. One end of the second bending adjustment wire 4073 is fixed to the adapter seat 4080, and the other end of the second bending adjustment wire 4073 passes through the fourth chamber and the second wire entry hole 4014 and is fixed to the second rotating disk 4072. The second rotating disk 4072 is configured to rotate synchronously with the second rotating shaft 4071 to tighten or loosen the second bending adjustment wire 4073.

The second bending adjustment structure 4070 also has two second rotating disks 4072 and two second bending adjustment wires 4073. A second projection and a second positioning bead (not shown) are also provided between the second rotating disk 4072 and the fixed block 4030. A second wire connecting rod 4074 fixedly connected to the second bending adjustment wire 4073 is also provided on the second rotating disk 4072, and a second bending adjustment cap 4075 is also provided corresponding to the second rotating shaft 4071. The second bending adjustment structure 4070 has the same structural principle as the first bending adjustment structure 4020, and can also realize bending functions in two opposite directions. Therefore, the second bending adjustment structure 4070 is not described in detail here. As shown in Figures 26 and 27, the first bending adjustment structure 4020 and the second bending adjustment structure 4070 can be staggered. This is to facilitate operation and simplify the bending adjustment device 4000. When the first bending adjustment structure 4020 and the second bending adjustment structure 4070 are staggered, an assembly plate 4032 is provided on the side of the fixed block 4030 facing the bending adjustment top cover 4091, and a second positioning bead is provided between the corresponding second rotating disk 4072 and the assembly plate 4032.

As shown in Figure 28 to Figure 35, the suture system also includes an exhaust device 5000, which is configured to exhaust the tube body in the suture system. The tube body in the suture system includes a main tube 4010, an inner tube 5030 and a plurality of suture needles 1030. The main tube 4010 includes a second chamber 4012 and a plurality of third chambers 4013. The plurality of third chambers 4013 are spaced around the axis of the second chamber 4012. The inner tube 5030 can be movably sleeved in the second chamber 4012, and the suture needle 1030 can be movably sleeved in the third chamber 4013. The exhaust device 5000 includes: a first exhaust structure 5010 and a second exhaust structure 5020.

The first exhaust structure 5010 includes an exhaust pipe 5011, a first knob 5012, a screw cap 5013 and a first sealing ring 5014. One end of the exhaust pipe 5011 is detachably sleeved with the first knob 5012, and the other end of the exhaust pipe 5011 is detachably sleeved with the screw cap 5013. The first sealing ring 5014 is located in the exhaust pipe 5011 and contacts the first knob 5012. A liquid injection port is provided on the screw cap 5013 or the exhaust pipe 5011 on the side of the first sealing ring 5014 away from the first knob 5012. The first knob 5012 is configured to allow the main tube 4010 to penetrate or pass through the exhaust pipe 5011. The first knob 5012 is also configured to squeeze the first sealing ring 5014 so that the first sealing ring 5014 and the outer periphery of the main tube 4010 are tightly matched in the radial direction. Alternatively, the first knob 5012 is also configured to be away from the first sealing ring 5014 so that the first sealing ring 5014 and the outer periphery of the main tube 4010 are spaced apart in the radial direction.

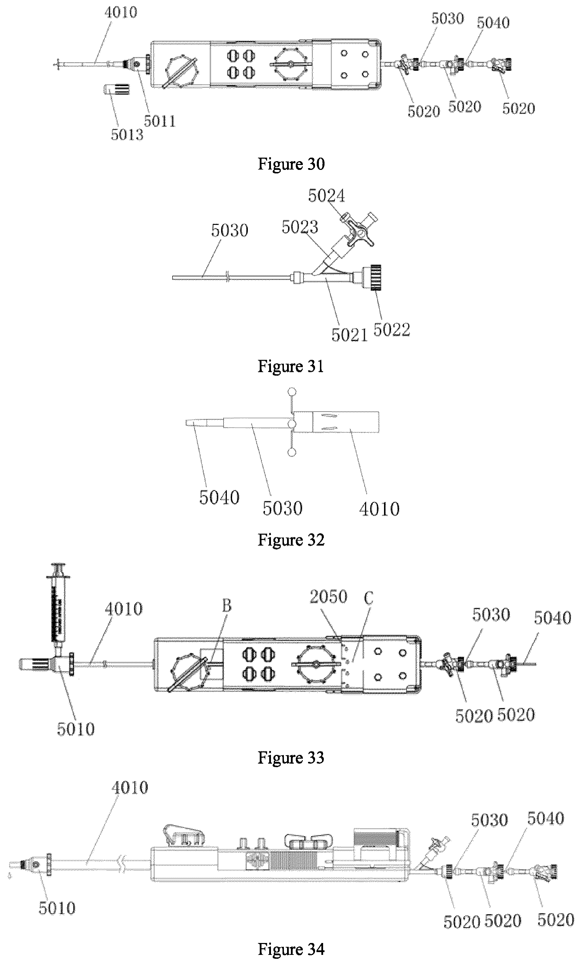

As shown in Figure 31, the second exhaust structure 5020 includes a first tubular object 5021, a second sealing ring (not shown) and a second knob 5022. One end of the first tubular object 5021 is sleeved with one end of the main tube 4010 away from the first exhaust structure 5010, and the other end of the first tubular object 5021 is sleeved with the second knob 5022. The second sealing ring is located in the first tubular object 5021 and contacts the second knob 5022. The second knob 5022 is configured to allow the inner tube 5030 to pass through and be placed in the main tube 4010. The second knob 5022 is also configured to squeeze the second sealing ring so that the second sealing ring and the outer periphery of the inner tube 5030 are tightly matched in the radial direction. Alternatively, the second knob 5022 is also configured to be away from the second sealing ring so that the second sealing ring and the outer periphery of the main tube 4010 are spaced apart in the radial direction.

When exhaust is required, the screw cap 5013 is connected to the exhaust pipe 5011, and liquid is injected into the exhaust pipe 5011 through the liquid injection port. The first sealing ring 5014 is tightly matched with the main tube 4010, and the second sealing ring and the inner tube 5030 are spaced apart.

As shown in Figures 28 and 29, specifically, a first channel 5011a and a second channel 5011b communicated in the axial direction of the exhaust pipe 5011 are provided. The radial dimension of the first channel 5011a is smaller than the radial dimension of the second channel 5011b. An internal thread is provided in the second channel 5011b, and the first knob 5012 is threadedly connected to the internal thread of the exhaust pipe 5011. A first sealing ring 5014 is placed at the end of the second channel 5011b close to the first channel 5011a. An external thread is provided on a section of the outer wall of the exhaust pipe 5011 away from the second channel 5011b, and the screw cap 5013 is threadedly connected to the external thread on the exhaust pipe 5011. A sealing ring 5015 is provided on the outer wall of the exhaust pipe 5011, and the sealing ring 5015 is located at one end of the external thread close to the second channel 5011b. When the screw cap 5013 and the exhaust pipe 5011 are threadedly connected, the two ends of the sealing ring 5015 are respectively in abutment with the exhaust pipe 5011 and the screw cap 5013. A liquid injection tube 5011c is provided on the outer wall of the exhaust pipe 5011, and a liquid injection channel 5011d is formed in the liquid injection tube 5011c. The end of the liquid injection channel 5011d close to the exhaust pipe 5011 is the so-called liquid injection port. The first exhaust structure 5010 also includes a receiving tube 5016, and the receiving tube 5016 is fixed in the first channel 5011a, and part of the receiving tube 5016 extends outside the first channel 5011a, that is, extends outside the exhaust pipe 5011. The receiving tube 5016 can temporarily store the front end of the main tube 4010, especially the front-end seat 4040 and the multiple positioning rods 4041 on the front-end seat 4040. The receiving tube 5016 temporarily receiving the main tube 4010, the front-end seat 4040 and the positioning rod 4041, as shown in Figure 35, inserts the positioning rod 4041, the front-end seat 4040 and the main tube 4010 into the sheath tube 5050 by the receiving tube 5016 sleeved with the tail end 10432 of the sheath tube 5050.

As shown in Figure 31, the second exhaust structure 5020 also includes a second tubular object 5023, and the second tubular object 5023 is tilted relative to the first tubular object 5021. The first tubular object 5021 and the second tubular object 5023 are similar to the tubular structure. The spaces in the first tubular object 5021 and the second tubular object 5023 are communicated. A valve 5024 is provided on the second tubular object 5023, and the communication between the space in the second tubular object 5023 and the outside is controlled by the valve 5024.

The specific situation of the tube body sleeve connection in the suture system is as follows: the suture system mainly includes a main tube 4010, an inner tube 5030, a reinforcing sheath core 5040, and a connecting tube 3020 connected to the capture net 3010. The reinforcing sheath core 5040 is a tube body structure, as shown in Figure 32, the inner tube 5030 is sleeved in the second chamber 4012 of the main tube 4010, and a portion of it is passed out. The reinforcing sheath core 5040 is sleeved in the inner tube 5030, and a portion of it is passed out. The end of the reinforcing sheath core 5040 is a tapered tube body, and the role of arranging the reinforcing sheath core 5040 is to guide the inner tube 5030. Firstly, the tapered tube body portion of the reinforcing sheath core 5040 passes through the atrial septum A1 of the foramen ovale A, by which the inner tube 5030 passes through the atrial septum A1 of the foramen ovale A; then, after the inner tube 5030 passes through the atrial septum A1 of the foramen ovale A to reach the left atrium A3, the reinforcing sheath core 5040 can be withdrawn, and the strip-shaped capture net 3010, through the connecting tube 3020, is passed through the inner tube 5030 and out to reach the left atrium A3. Based on this, four second exhaust structures 5020 in the embodiment of the present disclosure can be configured based on the sleeve connection of the tube body.

As shown in Figure 30, three second exhaust structures 5020 are provided from left to right, and the second exhaust structure 5020 on the left side is sealed and fixedly sleeved with the rear end of the main tube 4010 through the first tubular object 5021. The inner tube 5030 passes through the second knob 5022 in the second exhaust structure 5020 on the left side into the second chamber 4012 of the main tube 4010. The end of the inner tube 5030 away from the front end of the main tube 4010 is sealed and fixedly sleeved with the first tubular object 5021 in the middle second exhaust structure 5020, and the reinforcing sheath core 5040 penetrates into the inner tube 5030 through the second knob 5022 of the middle second exhaust structure 5020. The end of the reinforcing sheath core 5040 away from the front end of the main tube 4010 is sealed and fixedly sleeved with the first tubular object 5021 in the second exhaust structure 5020 on the right side. The fourth second exhaust structure 5020 is sleeved with the proximal end 3021 of the connecting tube 3020 in the suture grasping device 3000. Specifically, the end of the first tubular object 5021 in the fourth second exhaust structure 5020 away from the second knob 5022 is sealed and fixedly sleeved with the proximal end 3021 of the connecting tube 3020.

As shown in Figure 30, when exhaust is not performed, the screw cap 5013 in the first exhaust structure 5010 is removed, and the first knob 5012 is rotated so that the first sealing ring 5014 and the outer periphery of the main tube 4010 are spaced apart in the radial direction. The remaining first exhaust structures 5010 can be moved to the end of the main tube 4010 close to the bending adjustment handle 4090, and then the first sealing ring 5014 and the outer periphery of the main tube 4010 are tightly matched in the radial direction by the first knob 5012.

When exhaust is required, as shown in Figure 33, the first exhaust structure 5010 without the screw cap 5013 is moved to the front end of the main tube 4010 by operating the first knob 5012. At this time, the front end of the main tube 4010, the front-end seat 4040 and the positioning rod 4041 are all received in the receiving tube 5016, and the screw cap 5013 and the exhaust pipe 5011 are threadedly connected. The second knob 5022 of the second exhaust structure 5020 on the main tube 4010, the second knob 5022 of the second exhaust structure 5020 on the inner tube 5030, and the second knob 5022 of the second exhaust structure 5020 on the reinforcing sheath core 5040 are all in a loosened state. Taking the main tube 4010 as an example, when the second knob 5022 is in a loosened state, the second sealing rings in the second exhaust structure 5020 and the main tube 4010 are radially spaced apart. Physiological saline is injected into the exhaust pipe 5011 through the liquid injection channel 5011d, and the physiological saline flows from the gap between the main tube 4010 and the exhaust pipe 5011 to the side of the screw cap 5013, and then flows into the second chamber 4012, the third chamber 4013, the inner tube 5030, the reinforcing sheath core 5040 and the suture needle 1030 of the main tube 4010 through the end of the receiving tube 5016 away from the first knob 5012. As shown in Figure 33, a first visual window B is provided on the bending adjustment top cover 4091, through which the place where the suture needle 1030 penetrates the main tube 4010 can be seen, and a second visual window C is provided on the needle ejection top cover 1011, through which the end of the guide tube 2050 away from the suture needle 1030 can be seen. If water is seen flowing out of the first visual window B, it means that the third chamber 4013 containing the suture needle 1030 is successfully exhausted. If water is seen flowing out of the second visual window C, it means that the exhaust in the suture needle 1030 is successful. If water can be seen flowing out of the second knob 5022 corresponding to the main tube 4010, it means that the second chamber 4012 is successfully exhausted. If water can be seen flowing out of the second knob 5022 corresponding to the inner tube 5030, it means that the inner tube 5030 is successfully exhausted. If water can be seen flowing out of the second knob 5022 corresponding to the reinforcing sheath core 5040, it means that the reinforcing sheath core 5040 is successfully exhausted. In order to better ensure the exhaust effect, the structure shown in Figure 33 can be made lower on the left and higher on the right, wherein the exhausts of the capture net 3010 and the corresponding connecting tube 3020 can be performed separately. When the exhaust is completed, the second knob 5022 corresponding to the main tube 4010, the second knob 5022 corresponding to the inner tube 5030, the second knob 5022 corresponding to the reinforcing sheath core 5040, and the second knob 5022 corresponding to the connecting tube 3020 are all in a tightened state. Taking the main tube 4010 as an example, when the second knob 5022 is tightened, the outer periphery of the main tube 4010 and the second sealing ring are radially tightly matched to prevent external gas from entering the tube body to maintain the exhaust effect.

The four second exhaust structures 5020 can be the same, and adaptive adjustments can be made based on the different sizes of the corresponding tube bodies. The second tubular objects 5023 in the four second exhaust structures 5020 can be configured to be filled with contrast agents, and the contrast agents can be used to determine whether the corresponding tube body has moved to a suitable position. Taking the inner tube 5030 as an example, the second tubular object 5023 corresponding to the inner tube 5030 is filled with contrast agents, and the contrast agents flow forward along the inner tube 5030, that is, flow into the human body. When the contrast agents flow out from one end of the inner tube 5030 placed in the human body, the contrast agents can be seen, and then the current position of the inner tube 5030 can be determined by the contrast agents, so as to make timely adjustments when the device is abnormally operated.

As shown in Figure 34, in other embodiments, in addition to exhausting by inputting physiological saline into the liquid injection tube 5011c, the physiological saline can also be input into the second tubular object 5023 to exhaust. For example, when exhausting the main tube 4010 alone, the physiological saline can be input through the second tubular object 5023 corresponding to the main tube 4010. The second knobs 5022 in the plurality of second exhaust structures 5020 are all in a tightened state, and the screw caps 5013 are separated with the exhaust pipe 5011 at this time. If water can be seen flowing out at the receiving tube 5016, it means that the second chamber 4012 of the main tube 4010 is successfully exhausted.

As shown in Figure 35, during the use of the suture system, since the front-end seat 4040 and other structures in the receiving tube 5016 need to be taken out to facilitate the passage of the guide wire 5060, for example, the guide wire 5060 passes through the reinforcing sheath core 5040, the structure in the receiving tube 5016 needs to be exhausted again. At this time, physiological saline can be input into the liquid injection tube 5011c in the first exhaust structure 5010. If water is observed flowing out at the receiving tube 5016, it means that the structure at the receiving tube 5016 is successfully exhausted.

The suture system in the embodiment of the present disclosure, in addition to the needle ejection device 1000 and the suture control device 2000, can also include one or more of the suture grasping device 3000, the bending adjustment device 4000, and the exhaust device 5000, or even all of them. Among them, the suture grasping device 3000 is configured to grasp the suture 2010. The bending adjustment device 4000 is configured to bend the front end of the main tube 4010 containing the suture needle 1030. The exhaust device 5000 is configured to exhaust the suture system. Specifically, in the embodiment of the present disclosure, the suture system including the needle ejection device 1000, the suture control device 2000, the suture grasping device 3000, the bending adjustment device 4000 and the exhaust device 5000 is taken as an example to illustrate the use principle. The application of the suture system specifically takes the closure of the foramen ovale as an example. In addition to structural heart diseases, such as the closure of the foramen ovale, the atrial septal defect, and the ventricular septal defect, other fields that require suturing can also use the suture system in the present disclosure, such as the suture system of the embodiment of the present disclosure in the vascular suture operation.

The specific use principle of the suture system is as follows.

Step 1: As shown in Figures 29 and 33, the relevant tubes of the suture system are exhausted by injecting physiological saline into the liquid injection tube 5011c, and the second chamber 4012, the third chamber 4013, the suture needle 1030, the inner tube 5030 and the reinforcing sheath core 5040 on the main tube 4010 are exhausted at the same time. The specific implementation principle of exhaust and the operation at the time of exhaust and exhaust ending can be seen in the principle introduction of the exhaust device 5000 mentioned above, which will not be repeated here.

Step 2: As shown in Figure 37, the guide wire 5060 enters the left atrium A3.

Step 3: As shown in Figure 38, the sheath tube 5050 enters the right atrium A2 near the atrial septum A1 along the guide wire 5060.

Step 4: As shown in Figure 35, the front end of the suture system is exhausted, that is, the front end of the main tube 4010 and the front-end seat 4040 are exhausted. The guide wire 5060 is passed through the reinforcing sheath core 5040 until the guide wire 5060 passes through the second knob 5022 corresponding to the reinforcing sheath core 5040, and the front end of the main tube 4010 or the receiving tube 5016 is close to the tail of the sheath tube 5050. The front end of the main tube 4010, the front-end seat 4040 and the positioning rod 4041 are received into the receiving tube 5016, and the physiological saline is introduced into the liquid injection tube 5011c until the liquid flows out of the receiving tube 5016 (upward oblique port).

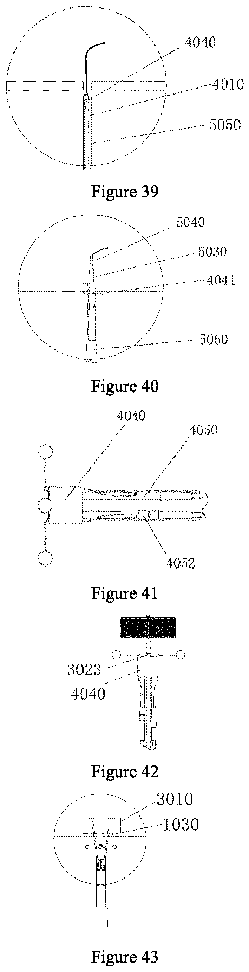

Step 5: As shown in Figure 39, the front end of the main tube 4010 enters the right atrium A2. Specifically, the front end of the main tube 4010 is inserted into the tail of the sheath tube 5050 through the receiving tube 5016 first, and then the main tube 4010 containing the inner tube 5030, the reinforcing sheath core 5040, and the suture needle 1030 is inserted into the sheath tube 5050.

Step 6: As shown in Figure 40, the sheath tube 5050 is withdrawn to open the multiple positioning rods 4041, and at the same time, the inner tube 5030 and the reinforcing sheath core 5040 are pushed to the front end of the inner tube 5030 beyond the atrial septum A1 for a distance, and the reinforcing sheath core 5040 itself exceeds the inner tube 5030 for a distance. The suture system is pushed (for example, by adjusting the bending adjustment handle 4090, the needle ejection handle 1010 or the suture handle 2030) to make the positioning rod 4041 close to the atrial septum A1. The positioning rod 4041 has elasticity and rigidity. After touching the atrial septum A1, the positioning rod can be squeezed and deformed by the atrial septum A1, so that the position where the positioning rod 4041 begins to deform can be determined from the image. At this position, the positioning rod 4041 is close to the atrial septum A1 and will not cause the atrial septum A1 to bear too much external force.

Step 7: The sheath tube 5050 and the inner tube 5030 are fixed, and the reinforcing sheath core 5040 and the guide wire 5060 are withdrawn from the body. After the reinforcing sheath core 5040 and the guide wire 5060 are withdrawn from the body, the reinforcing sheath core 5040 and the guide wire 5060 in Figure 40 disappear accordingly.

Step 8: The capture net 3010 is pushed. The sheath tube 5050 and the inner tube 5030 are fixed. The capture net 3010 is pushed to the left atrium A3 along the inner tube 5030 through the connecting tube 3020, and the state switching wire 3050 is pulled to make the capture net 3010 cylindrical (for example, as shown in Figure 18), and the inner tube 5030 is retracted into the main tube 4010, and the capture net 3010 is retracted until the lower end surface of the cylinder of the capture net 3010 is brought into close contact with the atrial septum A1 (in this state, the atrial septum A1 is clamped by the positioning rod 4041 and the capture net 3010).

Step 9: the bend is adjusted. The first bend adjustment cap 4026 and the second bend adjustment cap 4075 on the bend adjustment device 4000 are rotated to bend the first flexible section 4016 and the second flexible section 4017, as shown in Figure 27. During the bend adjustment process, the positioning rod 4041 is always kept close contact with the atrial septum A1, but the atrial septum A1 is not pulled or pushed to cause a large deformation of the atrial septum A1.

Step 10: As shown in Figure 41, the developing device rotates, until the four guide posts 4050 can be seen overlapping in pairs on the device screen (it can be determined that the developing plane of the device is parallel to the bending plane of the instrument, and the position of the front end of the main tube 4010 in the human body can be accurately determined to ensure the safety of the needle ejection). The developing device refers to the developing device used by the doctor during surgery, such as an X-ray developing device. Alternatively, as shown in Figure 25, the two developing parts 4052 on the fourth guide post 4050 are spaced apart in the axial direction, and the spacing between the two developing parts 4052 on the fourth guide post 4050 is not less than the axial length of the developing part 4052 on the first guide post 4050. After the developing device rotates, the three developing parts 4052 display three developing points on the developing device. When the three developing points are located in the same straight line, the needle can be safely ejected. This method is more convenient for doctors to determine that whether the developing plane of the device is parallel to the bending plane of the instrument.

Step 11: As shown in Figure 42, the needle is ejected at the same time. The connecting tube 3020 is sleeved with the developing ring 3023 on the outer wall close to the capture net 3010. The image is observed. When the lower edge line of the developing ring 3023 coincides with the upper edge line of the front-end seat 4040, the relative position of the capture device and the front-end seat 4040 is a preset relative position, and the needle can be safely ejected. The suture handle (the general term for the bending adjustment handle 4090, the needle ejection handle 1010 and the suture handle 2030) is fixed. For example, as shown in Figure 36, the pressing structures 1110 on both sides of the needle ejection top cover 1011 are pressed simultaneously and the needle ejection top cover 1011 is pushed forward to complete the needle ejection. The needle ejection distance is determined by combining the image to ensure that the longest suture needle 1030 does not exceed the top of the cylinder of the capture net 3010, ensuring the safety of the needle ejection. After the pressing structure 1110 is released, the position of the suture needle 1030 is fixed, and the simultaneous needle ejection is completed.

Step 12: As shown in Figure 43, a suture needle 1030 is adjusted individually (if the needles are ejected at the same time to meet the thread pushing requirement, this step can be omitted), the suture handle is fixed, the locking screw cap 1054 is loosened, and the suture needle 1030 that needs to be adjusted is determined according to the image. The push button 1100 corresponding to the suture needle 1030 is pushed or pulled to adjust the position of the suture needle 1030 relative to the capture net 3010 (by adjustment, the sharp part of the suture needle 1030 can be all inside the cylinder of the capture net 3010 to ensure smooth thread pushing). After the adjustment of the suture needle 1030 is completed, the locking screw cap 1054 is tightened. After the needle is adjusted individually, for example, the two needles in Figure 43 will be symmetrically distributed, and the lengths placed in the capture net 3010 will be equal.

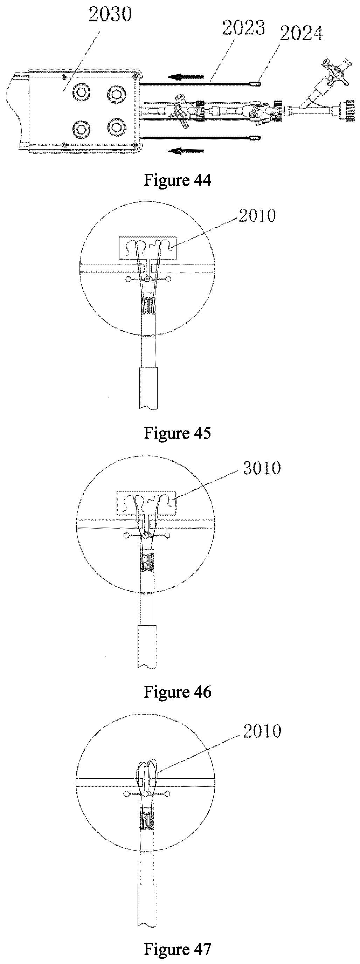

Step 13: As shown in Figure 44, the thread is pushed. The protective cover 2110 is removed, the thread return knob 2083 is pulled up, the thread return knob 2083 and the thread return shaft 2081 is disengaged, and the suture pushing assembly 2020 from both sides of the suture handle 2030 is removed. The movable second tube body 2023 on the suture pushing assembly 2020 is pushed to align it with the first tube body 2022 of the previous section, and then continues to be pushed forward (in the direction shown by the black arrow in the figure) until the second tube body 2023 on the suture pushing assembly 2020 contacts the limit joint 2024, and the suture pushing assembly 2020 stroke ends. After the thread pushing is completed, as shown in Figure 45, four sutures 2010 are arranged in the capture net 3010.

Step 14: As shown in Figure 45 to Figure 46, the suture needle 1030 is retracted. The first bending adjustment cap 4026 and the second bending adjustment cap 4075 are rotated to the initial position. The locking screw cap 1054 is loosened, the push button 1100 is moved to the far right, and the locking screw cap 1054 is tightened again. The pressing structure 1110 is pressed and the needle ejection top cover 1011 is moved to the far right to complete the recovery of the suture needle 1030.

Step 15: As shown in Figure 47 to Figure 48, the suture 2010 is captured. The suture handle is fixed, and the inner tube 5030 is pushed to the left atrium A3 beyond the atrial septum A1 for a distance and then fixed. The capture net 3010 is pulled backward. When the cylindrical shape of the capture net 3010 is retracted to about half, the suture pushing assembly 2020 is removed, and then the capture net 3010 is continued to be pulled until the capture net 3010 leaves the inner tube 5030. The suture 2010 captured by the capture net 3010 from the capture net 3010 is removed, and the quantity is confirmed.

Step 16: The suture 2010 is recovered (where it is not necessary under normal situations and retracting the suture 2010 is designed to deal with special situations; for example, if the suture 2010 cannot be completely pushed out of the suture needle 1030 or the suture 2010 is not captured, the suture 2010 needs to be retracted to ensure that the operation can be performed safely under any situations). The thread return knob 2083 is pressed and rotated to retract the suture 2010 into the suture needle 1030 for easy re-pushing.

Step 17: The instrument is withdrawn. The sheath 5050 is fixed, and the main tube 4010 and the inner tube 5030 are withdrawn from the body along the sheath 5050.

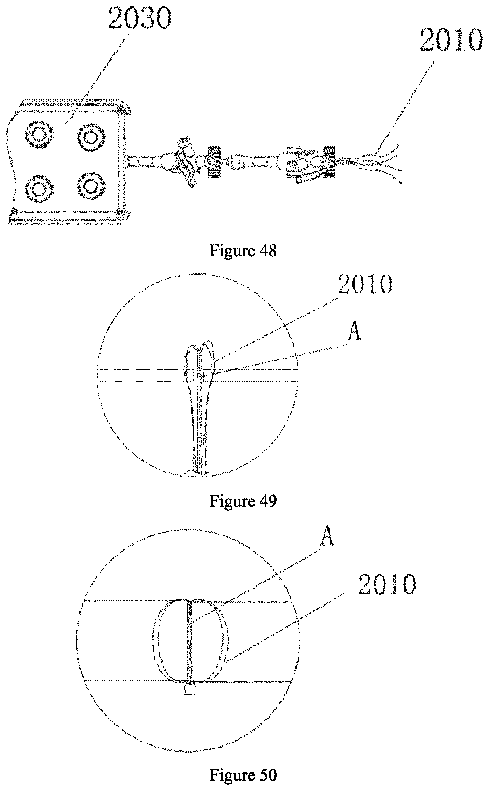

Step 18: As shown in Figures 49-50, the suture is cut. The suture 2010 is loaded into the cutting device, and the cutting device enters the right atrium A2 along the sheath 5050, the suture 2010 is tightened, and the suture 2010 after tying the suture 2010 is cut.

The above specific examples are used to illustrate the present disclosure, which are only configured to help understand the present disclosure and are not intended to limit the present disclosure. For those skilled in the art to which the present disclosure belongs, they can also make some simple deductions, deformations or substitutions based on the ideas of the present disclosure.

### INDUSTRIAL APPLICABILITY

In summary, the present disclosure provides a suture system that can realize both synchronous and separate needle ejection, effectively improve the efficiency and safety of the suture system, improve the puncture effect of the suture needle, and improve the success rate of the operation.

## Claims

1. A suture system, wherein the suture system comprises: a needle ejection device and a suture control device; the needle ejection device is configured to push out or retract the suture needle, and the suture control device is configured to push out or retract the suture;
the needle ejection device comprises a needle ejection handle, a guide member, a pushing body and a suture needle;
the needle ejection handle comprises a needle ejection top cover and a needle ejection bottom cover which are slidably connected;
the guide member is located in the needle ejection handle, and the guide member is fixedly connected to the needle ejection top cover;
the pushing body and the guide member are movably connected;
at least two the suture needles are configured, each suture needle is fixed to a pushing body, and the pushing bodies and the suture needles are arranged in a one-to-one correspondence; and
the needle ejection top cover is configured to slide relative to the needle ejection bottom cover to drive the guide member to move; and the pushing body is configured to move along the guide member to drive the connected suture needle to move.

2. The suture system according to claim 1, wherein the needle ejection device further comprises a locking member and at least two locking strips, wherein the locking strips are fixed to the pushing body; the locking member has a first state and a second state, and when the locking member is in the first state, the locking member is configured to lock at least two locking strips at the same time, and when the locking member is in the second state, the locking member is configured to release the locking of at least two locking strips.

3. The suture system according to claim 2, wherein the locking member comprises a locking post and a locking plate; a locking hole is provided on the needle ejection top cover, the locking plate is located in the needle ejection handle, a portion of the locking post passes through the locking hole and is threadedly connected to the locking plate, and the remaining portion of locking post is placed outside the needle ejection handle; when the locking member is in a first state, the locking plate abuts both of at least two locking strips, and when the locking member is in a second state, the locking plate and at least two locking strips are separated, and the locking post is configured to change a position of the locking plate relative to at least two locking strips by rotation.

4. The suture system according to claim 2 or 3, wherein the guide member comprises a first baffle, a second baffle, a third baffle and a conical block, and the conical block is fixed to a side of the first baffle away from the second baffle;
the pushing body comprises first pushing bodies and second pushing bodies, wherein two first pushing bodies and two second pushing bodies are configured, the suture needles are fixed to a same side of the first pushing body and the second pushing body close to the conical block, and an end of the suture needle away from the third baffle penetrates the conical block, and the conical block is configured to guide the suture needles into a main tube, and the locking strips are fixed on both of the first pushing body and the second pushing body;
first push shafts are fixed between the first baffle and the second baffle, the first push body is movably sleeved on the first push shaft, and at least one of the first push shafts is sleeved on each of the first push bodies;
second push shafts are fixed between the second baffle and the third baffle, the second push body is movably sleeved on the second push shaft, and at least one of the second push shafts is sleeved on each of the second push bodies; and
a plurality of adjustment holes are provided on the needle ejection top cover, and the first push body and the second push body are both fixed with push rods on sides facing the needle ejection top cover, and each of the push rods partially passes through one of the adjustment holes and is placed outside the needle ejection handle.

5. The suture system according to any one of claims 1 to 4, wherein pressing structures are symmetrically arranged on both side walls of the needle ejection top cover, the pressing structure comprises a pressing plate, an elastic sleeve and a clamping member, and sub-tooth portions and connecting rods are respectively arranged on both sides of the pressing plate, the sub-tooth portions are located on a side away from the needle ejection top cover, and the connecting rods are located on a side close to the needle ejection top cover, and multiple connecting rod are configured;
an end of each connecting rod away from the pressing plate passes through the side wall of the needle ejection top cover and is placed in the needle ejection top cover;
the elastic sleeve is arranged on each connecting rod between the pressing plate and the needle ejection top cover, the clamping member is fixed to the end of each connecting rod away from the pressing plate, and the elastic sleeve, the clamping member and the connecting rod are all arranged in a one-to-one correspondence; and
mother tooth portions are symmetrically arranged on the both side walls of the needle ejection bottom cover, the sub-tooth portions are configured to mesh with the mother tooth portions, and the number of teeth of the sub-tooth portions is less than the number of teeth of the mother tooth portions.

6. The suture system according to any one of claims 1 to 5, wherein the suture control device comprises a suture and a suture pushing assembly; an end of the suture is configured to penetrate into the suture needle, and the suture located in the suture needle is provided with an abutment head; the suture pushing assembly comprises a suture pushing wire, one end of the suture pushing wire abuts against the abutment head, another end of the suture pushing wire is located outside the needle ejection handle, and the suture pushing wire is configured to push the suture.

7. The suture system according to claim 6, wherein the suture pushing assembly further comprises a first tube body, a second tube body and a limit joint, the first tube body is fixedly sleeved on the suture pushing wire, the second tube body is movably sleeved on the suture pushing wire, one end of the suture pushing wire is a thread pushing end, and another end of the suture pushing wire is a free end, the first tube body is located on a side near the thread pushing end, the second tube body is located on a side near the free end, and the limit joint is fixedly connected to the free end.

8. The suture control device according to claim 7, wherein the suture control device further comprises a suture handle, an adapter joint and a guide tube, an adapter groove is provided outside the suture handle, the adapter joint has a first docking hole and a second docking hole communicated in an axial direction, the suture needle is arranged at one end of the adapter joint and the guide tube is arranged at the other end of the adapter joint, part of the suture needle is fixedly sleeved in the first docking hole, and part of the guide tube is fixedly sleeved in the second docking hole;
part of the first tube body close to the thread pushing end is capable of being movably placed in the guide tube, the second tube body and the limit joint are located outside the suture handle, and the second tube body is capable of being detachably fixed in the adapter groove; and
the suture, the suture pushing assembly, the adapter joint, the guide tube and the suture needle are arranged in a one-to-one correspondence; when the suture needs to be pushed out of the suture needle, the second tube body penetrates into the suture handle and abuts against the first tube body, and the thread pushing end abuts against the abutment head.

9. The suture system according to claim 8, wherein the suture handle comprises a suture top cover and a suture bottom cover; the suture control device further comprises a reel seat, a thread drum and a thread return shaft, which are located in the suture handle;
the reel seat is located at an end of the guide tube away from the adapter joint, the reel seat is provided with a plurality of reel holes on a side facing the suture top cover, and a plurality of thread pushing grooves are provided on the reel seat, the thread pushing grooves and the reel holes are arranged in a one-to-one correspondence, one end of the thread pushing groove is tangent to and communicated with the reel hole, and another end of the thread pushing groove extends to an end surface of the reel seat close to the adapter joint;
the thread drum is rotatably fixed in the reel hole, and the thread drum is wound with multiple turns of suture, and an end of the suture passes through the thread pushing groove and the guide tube into the suture needle;
the thread return shaft is fixedly connected to the thread drum, and the thread return shaft is configured to rotate the thread drum to perform thread return operation;
the suture top cover is made of transparent material, and the thread drum is provided with a marking position on a side facing the suture top cover, and the thread drum, the thread return shaft, the reel hole, the suture, the suture pushing assembly, the adapter joint, the guide tube and the suture needle are all arranged in a one-to-one correspondence; and
multiple guide tubes are configured, and the multiple guide tubes are spaced apart.

10. The suture system according to any one of claims 1 to 9, wherein the suture system further comprises a suture grasping device, and the suture grasping device comprises:
a capture net having a contracted state and an expanded state, the capture net being strip-shaped in the contracted state and post-shaped in the expanded state;
a connecting tube having a proximal end and a distal end, the distal end being connected to one end of the capture net;
a reinforcing member, the reinforcing member being connected to another end of the capture net;
a support tube communicating with the connecting tube, one end of the support tube being connected to the distal end, another end of the support tube being a free end, and the support tube being located in the capture net, the free end of the support tube being configured to abut against or be sleeved with the reinforcing member, wherein the capture net is in the expanded state; and
a state switching wire, one end of the state switching wire passing through the connecting tube and the support tube into the capture net and being connected to the reinforcing member, another end of the state switching wire being located outside the connecting tube, the state switching wire being configured to move along an axial direction of the connecting tube to switch a state of the capture net.

11. The suture system according to any one of claims 1 to 10, wherein the suture system further comprises a bending adjustment device, and the bending adjustment device comprises a main tube and a first bending adjustment structure;
the main tube has a front end and a rear end, a first chamber is provided in the main tube, and a first wire entry hole is provided on a side wall of the main tube between the front end and the rear end, and the first wire entry hole is communicated to the first chamber;
the first bending adjustment structure comprises a first rotating disk, a first rotating shaft and a first bending adjustment wire; one end of the first bending adjustment wire is fixed to the front end of the main tube, and another end of the first bending adjustment wire passes through the first chamber and the first wire entry hole and is fixed to the first rotating disk; and
the first rotating disk and the first rotating shaft are fixedly connected, and the first rotating disk is configured to rotate synchronously with the first rotating shaft to tighten or loosen the first bending adjustment wire.

12. The suture system according to claim 11, wherein the bending adjustment device further comprises a fixed block and a first positioning bead;
the first rotating shaft and the fixed block are rotatably connected, two first rotating disks are configured, and the two first rotating disks are located at both ends of the fixed block;
a plurality of first projections are arranged on one end surface of the first rotating disk facing the fixed block, and the plurality of first projections are arranged in an array around an axis of the first rotating disk, and a first clamping space is formed between adjacent first projections; a plurality of first positioning beads are fixed on a side of the fixed block facing the first rotating disk, and beads of the first positioning beads are clamped in the first clamping space, and the beads of the first positioning beads are able to be clamped in different first clamping spaces when the first rotating shaft rotates; and the first rotating disk, the first bending adjustment wire and the first wire feeding hole are all arranged in a one-to-one correspondence; and
ends of the two first bending adjustment wires away from the front end of the main tube are distributed on both sides of the axis of the main tube, so that when the first rotating shaft rotates, any one of the two first bending adjustment wires is in a tightened state, and another of the two first bending adjustment wires is in a relaxed state.

13. The suture system according to claim 12, wherein the bending adjustment device further comprises a second bending adjustment structure, the second bending adjustment structure comprises a second rotating shaft, a second rotating disk and a second bending adjustment wire;
from a front end side of the main tube to a rear end side of the main tube, the main tube comprises a needle ejection section, a first flexible section, a second flexible section and a main section connected in sequence, the first bending adjustment structure is configured to bend the first flexible section, the second bending adjustment structure is configured to bend the second flexible section, and bending directions of the second bending adjustment structure and the first bending adjustment structure are opposite;
an adapter seat is fixed between the first flexible section and the second flexible section; a fourth chamber is provided in the main tube; a second wire entry hole is provided on a side wall of the main tube between the front end and the rear end, and the second wire entry hole is communicated to the fourth chamber; and
the second rotating shaft is fixedly connected to the second rotating disk; one end of the second bending adjustment wire is fixed to the adapter seat, and another end of the second bending adjustment wire passes through the fourth chamber and the second wire entry hole and is fixed to the second rotating disk; and the second rotating disk is configured to rotate synchronously with the second rotating shaft to tighten or loosen the second bending adjustment wire.

14. The suture system according to any one of claims 11 to 13, wherein the bending adjustment device further comprises a front-end seat, the front-end seat is connected to the front end of the main tube;
the front-end seat is provided with a wire threading hole, the first bending adjustment wire passes through the wire threading hole to become a double-strand bending adjustment wire, one end of the double-strand bending adjustment wire is fixed to the wire threading hole, and another end of the double-strand bending adjustment wire passes through the first chamber and is fixed to the first rotating disk; or, the bending adjustment device further comprises an adapter post, one end of the adapter post is fixed to one end of the front-end seat close to the main tube, the adapter post is fixedly connected to the first bending adjustment wire, and the adapter post is sleeved and fixed in the first chamber.

15. The suture system according to claim 14, wherein the main tube is further provided with a second chamber and a plurality of third chambers, the second chamber is configured to allow the inner tube to pass through, and the third chambers are configured to allow the suture needles to pass through; the bending adjustment device further comprises a plurality of guide posts, one end of the guide post is fixed to an end of the front-end seat close to the main tube, and another end of the guide post is placed in the third chamber, and the guide post is configured to allow the suture needle to pass out of the main tube; at least two groups of developing parts are provided on the plurality of guide posts, and the developing parts are configured to distinguish the guide posts at different positions.

16. The suture system according to claim 15, wherein a plurality of the third chambers are arranged in an array around the axis of the main tube, four third chambers are configured, the third chambers, the guide posts and the suture needles are arranged in a one-to-one correspondence; developing parts are respectively arranged on the outer side walls of any two adjacent guide posts among the four guide posts, any one of the two guide posts with the developing parts has one developing part thereon, and another one of the two guide posts with the developing parts has two developing parts thereon, a developing distance is formed between the two developing parts on the same guide post, and the developing distance is greater than an axial length of the developing part; and at least one first bending adjustment wire is arranged between the two guide posts with the developing parts.

17. The suture system according to any one of claims 1 to 16, wherein the suture system further comprises an exhaust device configured to exhaust tube bodies in the suture system, wherein the tube bodies in the suture system comprise a main tube, an inner tube and a plurality of the suture needles, the main tube comprises a second chamber and a plurality of third chambers, the plurality of the third chambers are spaced around an axis of the second chamber, the inner tube is able to be movably sleeved in the second chamber, and the suture needle is able to be movably sleeved in the third chamber; and the exhaust device comprises: a first exhaust structure and a second exhaust structure;
the first exhaust structure comprises an exhaust pipe, a first knob, a screw cap and a first sealing ring, one end of the exhaust pipe is detachably sleeved with the first knob, and another end of the exhaust pipe is detachably sleeved with the screw cap, the first sealing ring is located in the exhaust pipe and contacts the first knob, and a liquid injection port is provided on the screw cap or the exhaust pipe on a side of the first sealing ring away from the first knob; the first knob is configured to allow the main tube to penetrate or pass through the exhaust pipe, and the first knob is further configured to squeeze the first sealing ring so that the first sealing ring and an outer periphery of the main tube are tightly matched, or the first knob is further configured to be away from the first sealing ring so that the first sealing ring and the outer periphery of the main tube are spaced apart;
the second exhaust structure comprises a first tubular object, a second sealing ring and a second knob, one end of the first tubular is sleeved with one end of the main tube away from the first exhaust structure, and another end of the first tubular object is sleeved with the second knob, the second sealing ring is located in the first tubular object and contacts the second knob; the second knob is configured to allow the inner tube to pass through and be placed in the main tube, and the second knob is further configured to squeeze the second sealing ring so that the second sealing ring and the outer periphery of the inner tube are tightly matched, or the second knob is further configured to be away from the second sealing ring so that the second sealing ring and the outer periphery of the main tube are spaced apart; and
when exhaust is required, the screw cap is connected to the exhaust pipe, and liquid is injected into the exhaust pipe through the liquid injection port, the first sealing ring and the main tube are tightly matched, and the second sealing ring and the inner tube are spaced apart.

18. The suture system according to any one of claims 1 to 17, wherein the suture system further comprises a suture grasping device, a bending adjustment device, and an exhaust device; the suture grasping device is configured to grasp the suture, the bending adjustment device is configured to bend a front end of the main tube accommodating the suture needle, and the exhaust device is configured to exhaust the suture system.
